# EUROPEAN PATENT APPLICATION

(11) **EP 2 818 172 A1**
(43) Date of publication of application: **31.12.2014**
(21) Application number: 13752554.9
(22) Date of filing: 21.02.2013
(51) Int. Cl.: A61K 31/728, A23L 1/30, A61K 8/60, A61K 8/73, A61K 31/7016, A61K 31/702, A61P 3/04, A61P 3/06, A61P 3/10, A61P 9/10, A61P 9/12, A61P 11/00, A61P 13/12, A61P 17/16, A61P 19/02, A61P 21/00, A61P 25/00, A61P 25/28, A61P 29/00, A61P 35/00

(54) **TLR4 AGENT, TISSUE HOMEOSTASIS AGENT, HEPATOCYTE GROWTH FACTOR INDUCER, TISSUE REPAIRING AGENT, AND SIRTUIN INDUCER HAVING HYALURONIC ACID FRAGMENTS AS ACTIVE INGREDIENTS THEREOF**

(30) Priority: 22.02.2012 JP 2012035743; 22.02.2012 JP 2012035745; 21.09.2012 JP 2012208055
(71) Applicant: Hyaluronan Research Institute, Inc., Tokyo 180-0003 (JP)
(72) Inventor: ASARI Akira, Musashino-shi Tokyo 180-0003 (JP); CODEE Jeroen, 2025 Haarlem (NL)
(74) Representative: Denison, Christopher Marcus
(86) International application number: PCT/JP2013/054330
(87) International publication number: WO 2013/125634

(57) **Abstract**

Provided are a sirtuin inducer, a tissue repairing agent, a hepatocyte growth factor inducer, a tissue homeostasis maintenance agent, and a TLR4 agonist, which utilize a hyaluronic acid fragment as an active ingredient.

A hyaluronic acid fragment selected from sizes of 2 to 20 sugar units, a pharmaceutically acceptable salt thereof, or pharmaceutically acceptable solvates thereof, is employed as the active ingredient for the sirtuin inducer, tissue repairing agent, hepatocyte growth factor inducer, tissue homeostasis maintenance agent, or TLR4 agonist. The hyaluronic acid fragment is preferably selected from sizes of 3 to 10 sugar units, and more preferably selected from a size of 3 or 4 sugar units.

## Description

### TECHNICAL FIELD

The present invention relates to a sirtuin inducer, a tissue repairing agent, a hepatocyte growth factor inducer, a tissue homeostasis maintenance agent, and a TLR4 agonist, which utilize a hyaluronic acid fragment as an active ingredient.

### BACKGROUND ART

Hyaluronic acid is known as a natural polymer that is a constituent of the extracellular matrix in joints, skin, brain, and the like, and has been employed in the past as a moisturizing ingredient in pharmaceuticals and cosmetics.

Hyaluronic acid is a polysaccharide of linked disaccharide units of D-glucuronic acid and N-acetyl-D-glucosamine, and is classified, according to molecular weight, as high-molecular weight hyaluronic acid, low-molecular weight hyaluronic acid, or hyaluronic acid fragments. The molecular weight of high-molecular weight hyaluronic acid is typically on the order of from 100,000 to 1,000,000. The molecular weight of low-molecular weight hyaluronic acid is on the order of from 10,000 to 100,000. The molecular weight of hyaluronic acid fragments is on the order of from 400 to 10,000 (Non-patent document 1). Hyaluronic acid has various types of bioactivity in vivo, the bioactivity thereof differing to a great extent depending on molecular weight (Non-patent document 2).

### [Related Art Documents]

### [Non-patent Documents]

[Non-patent Document 1] Science, Vol. 228, pp. 1324-1326 (1985)
[Non-patent Document 2] Medical Application of Hyaluronan, Akira Asari, Chapter 21, Chemistry and Biology of Hyaluronan, Elsevier, ed.: Garg, HG and Hales CA, pp. 457-473 (2004)

### DISCLOSURE OF THE INVENTION

### [Problems to Be Solved by the Invention]

The bioactivity of hyaluronic acid fragments in particular is thought to be very different from that of high-molecular weight hyaluronic acid and low-molecular weight hyaluronic acid. However, their function and utility are still poorly understood.

An object of the present invention is to provide a sirtuin inducer, a tissue repairing agent, a hepatocyte growth factor inducer, a tissue homeostasis maintenance agent, and a TLR4 agonist, which utilize a hyaluronic acid fragment.

### [Means to Solve the Problems]

The inventors perfected the present invention as a result of painstaking research directed to achieving the aforementioned object. Specifically, the present invention is as follows.
(1) A sirtuin inducer, characterized by containing as an active ingredient a hyaluronic acid fragment selected from sizes of 2 to 20 sugar units, a pharmaceutically acceptable salt thereof, or a pharmaceutically acceptable solvate thereof.
(2) The sirtuin inducer according to the aforementioned (1), wherein the hyaluronic acid fragment is selected from sizes of 3 to 10 sugar units.
(3) The sirtuin inducer according to the aforementioned (1), wherein the hyaluronic acid fragment is selected from a size of 3 or 4 sugar units.
(4) The sirtuin inducer according to any of the aforementioned (1) to (3), for use in treatment and/or prevention of heart disease, arterial sclerosis, osteoporosis, inflammatory bowel disease, dementia, apoplexy, metabolic syndrome, cancer, pulmonary disease, renal disease, diabetes, osteoarthritis, rheumatism, progeria, radiation injury, muscle disorder, brain development disorder, neurological illness, hypertension, or obesity, or to inhibit aging.
(5) The sirtuin inducer according to any of the aforementioned (1) to (3), employed in the form of a pharmaceutical, functional food product, or cosmetic.
(6) A tissue repairing agent characterized by containing as an active ingredient a hyaluronic acid fragment selected from sizes of 2 to 20 sugar units, a pharmaceutically acceptable salt thereof, or a pharmaceutically acceptable solvate thereof.
(7) The tissue repairing agent according to the aforementioned (6), wherein the hyaluronic acid fragment is selected from sizes of 3 to 10 sugar units.
(8) The tissue repairing agent according to the aforementioned (6), wherein the hyaluronic acid fragment is selected from a size of 3 or 4 sugar units.
(9) The tissue repairing agent according to any of the aforementioned (6) to (8), for use in repair of the cornea, dermis, epithelial tissue, neurological tissue, or cardiac muscle tissue.
(10) The tissue repairing agent according to any of the aforementioned (6) to (8), employed in the form of a pharmaceutical, functional food product, or cosmetic.
(11) A hepatocyte growth factor inducer characterized by containing as an active ingredient a hyaluronic acid fragment selected from sizes of 2 to 20 sugar units, a pharmaceutically acceptable salt thereof, or a pharmaceutically acceptable solvate thereof.
(12) The hepatocyte growth factor inducer according to the aforementioned (11), wherein the hyaluronic acid fragment is selected from sizes of 3 to 10 sugar units.
(13) The hepatocyte growth factor inducer according to the aforementioned (11), wherein the hyaluronic acid fragment is selected from a size of 3 or 4 sugar units.
(14) The hepatocyte growth factor inducer according to any of the aforementioned (11) to (13), for use in treatment and/or prevention of liver damage, or in organ preservation.
(15) The hepatocyte growth factor inducer according to any of the aforementioned (11) to (13), employed in the form of a pharmaceutical, functional food product, or cosmetic.
(16) A tissue homeostasis maintenance agent characterized by containing as an active ingredient a hyaluronic acid fragment selected from sizes of 2 to 20 sugar units, a pharmaceutically acceptable salt thereof, or a pharmaceutically acceptable solvate thereof.
(17) The tissue homeostasis maintenance agent according to the aforementioned (16), wherein the hyaluronic acid fragment is selected from sizes of 3 to 10 sugar units.
(18) The tissue homeostasis maintenance agent according to the aforementioned (16), wherein the hyaluronic acid fragment is selected from a size of 3 or 4 sugar units.
(19) The tissue homeostasis maintenance agent according to any of the aforementioned (16) to (18), for use in maintaining stemness and inducing differentiation of cells in tissue.
(20) The tissue homeostasis maintenance agent according to any of the aforementioned (16) to (18), employed in the form of a pharmaceutical, functional food product, or cosmetic.
(21) A TLR4 agonist characterized by containing as an active ingredient a hyaluronic acid fragment selected from sizes of 2 to 20 sugar units, a pharmaceutically acceptable salt thereof, or a pharmaceutically acceptable solvate thereof.
(22) The TLR4 agonist according to the aforementioned (21), wherein the hyaluronic acid fragment is selected from sizes of 3 to 10 sugar units.
(23) The TLR4 agonist according to the aforementioned (21), wherein the hyaluronic acid fragment is selected from a size of 3 or 4 sugar units.
(24) The TLR4 agonist according to any of the aforementioned (21) to (23), for use in treatment and/or prevention of disease in the cornea, dermis, liver, neurological tissue, kidney, lung, heart, skeletal muscles, pancreas, or blood vessels.
(25) The TLR4 agonist according to any of the aforementioned (21) to (23), employed in the form of a pharmaceutical, functional food product, or cosmetic.

### [Advantageous Effects of the Invention]

The sirtuin inducer according to the present invention contains, as an active ingredient, a hyaluronic acid fragment selected from sizes of 2 to 20 sugar units, a pharmaceutically acceptable salt thereof, or a pharmaceutically acceptable solvate thereof, and therefore can promote expression of sirtuin proteins. The agent is therefore effectively employed, for example, in treatment and/or prevention of heart disease, arterial sclerosis, osteoporosis, inflammatory bowel disease, dementia, apoplexy, metabolic syndrome, cancer, pulmonary disease, renal disease, diabetes, osteoarthritis, rheumatism, progeria, radiation injury, muscle disorder, brain development disorder, neurological illness, hypertension, or obesity, or to inhibit aging.

The tissue repairing agent according to the present invention contains, as an active ingredient, a hyaluronic acid fragment selected from sizes of 2 to 20 sugar units, a pharmaceutically acceptable salt thereof, or a pharmaceutically acceptable solvate thereof, and therefore can repair tissue. The agent is therefore effectively employed, for example, in repair of the cornea, dermis, epithelial tissue, neurological tissue, or cardiac muscle tissue.

The hepatocyte growth factor inducer according to the present invention contains, as an active ingredient, a hyaluronic acid fragment selected from sizes of 2 to 20 sugar units, a pharmaceutically acceptable salt thereof, or a pharmaceutically acceptable solvate thereof, and therefore can promote expression of hepatocyte growth factor. The agent is therefore effectively employed, for example, in treatment and/or prevention of liver damage, or for organ preservation.

The tissue homeostasis maintenance agent according to the present invention contains, as an active ingredient, a hyaluronic acid fragment selected from sizes of 2 to 20 sugar units, a pharmaceutically acceptable salt thereof, or a pharmaceutically acceptable solvate thereof, and therefore can maintain homeostasis of tissue. The agent is therefore effectively employed, for example, for maintaining stemness and inducing differentiation of cells in tissue.

The TLR4 agonist according to the present invention contains, as an active ingredient, a hyaluronic acid fragment selected from sizes of 2 to 20 sugar units, a pharmaceutically acceptable salt thereof, or a pharmaceutically acceptable solvate thereof, and therefore exhibits action via TLR4. The agent is therefore effectively employed, for example, in treatment and/or prevention of disease in the cornea, dermis, liver, neurological tissue, kidney, lung, heart, skeletal muscles, pancreas, or blood vessels.

### BRIEF DESCRIPTION OF THE DRAWINGS

FIG. 1 shows photographs of stained images of human epidermal keratinocytes stained with anti human SIRT1 antibody in Test Example 1;
FIG. 2 is a graph showing results for the expression level of sirtuin proteins in Test Example 1;
FIG. 3 is a graph showing results for sirtuin enzymatic activity (sirt1 enzymatic activity) in cell solution in Test Example 2;
FIG. 4 shows photographs of stained images of human epidermal keratinocytes stained with anti human SIRT1 antibody in Test Example 3;
FIG. 5 is a graph showing results for change in size of human epidermal keratinocytes in Test Example 3;
FIG. 6 shows a photograph of a stained image of human epidermal keratinocytes stained with anti human SIRT1 antibody in Test Example 4;
FIG. 7 is a graph showing results of cell count, per unit of visual field area, of cells during division, the cells being smaller in form than other cells, and having higher expression of sirtuin proteins, in Test Example 4;
FIG. 8 is a graph showing results of cell count, per unit of visual field area, of cells during division, the cells being smaller in form than other cells, and having higher expression of sirtuin proteins, in Test Example 5;
FIG. 9 is a graph showing results of cell count, per unit of visual field area, of cells during division, the cells being smaller in form than other cells, and having higher expression of sirtuin proteins, in Test Example 6;
FIG. 10 shows photographs of stained images of human neural stem cells (Gibco human neural stem cells (H9-derived)) double-stained with anti human Nestin antibody and anti human tubulin antibody, in Test Example 7;
FIG. 11 is a graph showing results of the sirtuin protein expression level in Test Example 8;
FIG. 12 shows photographs of stained images of human large intestine epithelial cells (HT29 cells) stained with anti NF-kappaB antibody in Test Example 9;
FIG. 13 is a graph showing results of the anti NF-kappaB antibody expression level in Test Example 9;
FIG. 14 is a graph showing results of the anti NF-kappaB antibody expression level in Test Example 10;
FIG. 15 shows images of the condition of the cornea in the eye of a rabbit, 24 hours after inflicting injury to the cornea in Test Example 11;
FIG. 16 shows a graph of the surface area of the wound region, 24 hours after inflicting injury to the cornea in Test Example 11;
FIG. 17 shows images of the condition of the cornea in the eye of a rabbit, 48 hours after inflicting injury to the cornea in Test Example 11;
FIG. 18 shows a graph of the surface area of the wound region, 48 hours after inflicting injury to the cornea in Test Example 11;
FIG. 19 shows hematoxylin-eosin stains of corneal tissue of the eye of a rabbit, 33 hours after inflicting injury to the cornea in Test Example 12;
FIG. 20 shows photomicrographs of tissue slices of untreated liver prior to preservation, and of liver after being preserved in organ preservation solution, in Test Example 14;
FIG. 21 is a graph showing results for bleb (bubble) count per unit of visual field area in Test Example 14;
FIG. 22 is a graph showing results of blood GOT level in Test Example 15;
FIG. 23 is a graph showing results of age-inhibiting effect of hyaluronic acid fragments on human large intestine epithelial cells (HT29 cells) subjected to induced aging in Test Example 16;
FIG. 24 is a graph showing results of age-inhibiting effect of hyaluronic acid fragments on human large intestine epithelial cells (HT29 cells) subjected to induced aging in Test Example 17;
FIG. 25 is a graph showing results of age-inhibiting effect of hyaluronic acid fragments on human large intestine epithelial cells (HT29 cells) subjected to induced aging in Test Example 18;
FIG. 26 is a graph showing results of age-inhibiting effect of hyaluronic acid fragments on human oral epithelial cells (Ca9-22 cells) subjected to induced aging in Test Example 19;
FIG. 27 is a graph showing results of age-inhibiting effect of hyaluronic acid fragments on human neural stem cells (Gibco human neural stem cells (H9-derived)) subjected to induced aging in Test Example 20;
FIG. 28 is a graph showing results of age-inhibiting effect of hyaluronic acid fragments on rat bone marrow mesenchymal stem cells subjected to induced aging in Test Example 21;
FIG. 29 is a graph showing results for the keratinocyte marker Keratin K14 detected in human epidermal keratinocytes in Test Example 22; and
FIG. 30 is a graph showing results for the keratinocyte marker Filaggrin detected in human epidermal keratinocytes in Test Example 23.

### MODE FOR CARRYING OUT THE INVENTION

### (Hyaluronic acid fragments)

The hyaluronic acid fragments used in the present invention refer to those having sizes of 2 to 20 sugar units, and including at least disaccharide units of glucoside-linked D-glucuronic acid and N-acetyl-D-glucosamine, which make up the constituent units of hyaluronic acid. Specifically, the hyaluronic acid fragments are those having a structure represented by the following formula (1), formula (2), formula (3), or formula (4).

GlcA(-GlcNAc-GlcA)ₙ-GlcNAc (1)

(In formula (1), "GlcA" indicates a glucuronic acid residue, "GlcNAc" indicates an N-acetylglucosamine residue, "-" indicates a glucoside linkage, and "n" indicates an integer from 0 to 9.)

GlcA(-GlcNAc-GlcA)ₙ (2)

(In formula (2), "GlcA" indicates a glucuronic acid residue, "GlcNAc" indicates an N-acetylglucosamine residue, "-" indicates a glucoside linkage, and "n" indicates an integer from 0 to 9.)

GlcNAc(-GlcA-GlcNAc)ₙ-GlcA (3)

(In formula (3), "GlcA" indicates a glucuronic acid residue, "GlcNAc" indicates an N-acetylglucosamine residue, "-" indicates a glucoside linkage, and "n" indicates an integer from 0 to 9.)

GlcNAc(-GlcA-GlcNAc)ₙ (4)

(In formula (4), "GlcA" indicates a glucuronic acid residue, "GlcNAc" indicates an N-acetylglucosamine residue, "-" indicates a glucoside linkage, and "n" indicates an integer from 0 to 9.)

In the aforementioned formulas, the glucoside linkage in GlcA-GlcNAc is preferably the β1→3 linkage, and the glucoside linkage in GlcNAc-GlcA is preferably the β1→4 linkage.

Provided that a hyaluronic acid fragment used in the present invention has the aforedescribed basic structure, there are no particular limitations as to modification of the sugar moieties. For example, some or all of the hydroxyl groups of the sugar units may be subjected to esterification, etherification, or the like. Some or all of the carboxyl groups of glucuronic acid may be subjected to esterification, amidation, or the like. Sugar units located at non-reducing terminals may be saturated sugars (those in which the carbon-carbon bonds within monosaccharides include no double bonds) or unsaturated sugars (those in which the carbon-carbon bonds within monosaccharides include double bonds).

Because the size of the hyaluronic acid fragments is 2 to 20 sugar units, the fragments readily permeate into tissue and cells. Such low viscosity is suited to coating with good efficiency. The size is more preferably from 3 to 10 sugar units. In so doing, the fragments more readily permeate into tissue and cells. Moreover, such low viscosity is even more suited to coating with good efficiency. The size is most preferably 3 or 4 sugar units. In so doing, in addition to the aforedescribed effects, the effects with respect to tissues and cells are markedly better, as compared with other sizes.

In the present invention, the hyaluronic acid fragment can be employed in the form of pharmaceutically acceptable salts. As such salts, there can be cited alkali metal salts such as sodium salts, potassium salts, and the like. Besides anhydrous salts, hydrate salts may be included as well. In some instances, the salts may derive from the starting material, and in others introduced during preparation of the hyaluronic acid fragments. These salts undergo dissociation, for example, in solution, in vivo, or the like, to function in the same way as the hyaluronic acid fragment.

In the present invention, the hyaluronic acid fragment can be employed in the form of pharmaceutically acceptable solvates. Hydrates can be cited as an example of solvates. Hydrates may form, for example, when fragments are left in the air, are recrystallized, absorb moisture, or have adsorbed water adhering thereto. In some instances, the solvates introduced during preparation of the hyaluronic acid fragment. These solvates undergo dissociation, for example, in solution, in vivo, or the like, to function in the same way as the hyaluronic acid fragment.

The aforedescribed hyaluronic acid fragment, a pharmaceutically acceptable salt thereof, and a pharmaceutically acceptable solvate thereof are not limited solely to individual use, and multiple types (two or more) may be used together. That is, the hyaluronic acid fragments are not limited to a single size and molecular structure, and those of different size and molecular structure may be employed concomitantly; compounds containing multiple hyaluronic acid fragments of different size and molecular structure may be employed as well. With regard to salts and solvates as well, there is no limitation to a single type, and multiple different types may be employed concomitantly, or compounds containing multiple different types may be employed.

### (Manufacturing method of hyaluronic acid fragments)

The hyaluronic acid fragment used in the present invention may be derived from natural sources such as animals or the like, obtained through microbial culture, or synthesized chemically or enzymatically. In preferred practice, the hyaluronic acid fragment is manufactured by subjecting hyaluronic acid, which can be obtained through known extraction processes, for example, from biological tissues such as cock's comb, umbilicus, skin, joint fluid, or the like, to decomposition treatment such as enzymatic decomposition, acid decomposition, base decomposition, thermal decomposition, ultrasound treatment, or the like; and the hyaluronic acid fragment then purified by known purification methods. In similar fashion, hyaluronic acid produced by fermentation methods employing lactobacilli, Streptococcus bacteria, or the like can be manufactured as a starting material. Further, commercially available, high-molecular weight hyaluronic acid may be selected as a starting material.

In the case of manufacture of the hyaluronic acid fragment through an enzymatic decomposition method, manufacture can take place in accordance with the method disclosed in WO 2002/4471 or in Glycobiology, 2002, Vol. 12, No. 7, pp. 421-426. A high-molecular weight hyaluronic acid salt is dissolved in a buffer solution (for example, 0.1 M phosphate buffer solution) to a solution pH of about 5, adding hyaluronidase to bring about hydrolysis. The temperature for hydrolysis is preferably about 37°C, with the reaction taking place for about 1 to 24 hours. Following the reaction, the supernatant is recovered through centrifugal separation at 10,000 rpm, and the supernatant is fractionated through an ion-exchange column or the like, to obtain hyaluronic acid fragments of specific sizes.

In the case of manufacture of the hyaluronic acid fragment through an acid hydrolysis method, a high-molecular weight hyaluronic acid salt is reacted in an aqueous solution of 50% concentrated hydrochloric acid, for about 1 to 5 hours between 40°C and reflux temperature. Following the reaction, hyaluronic acid fragments of specific sizes are obtained through fractionation in an ion-exchange column or the like. Alternatively, hydrolysis can be brought about under milder conditions, through heating in a solution of DMSO, using about 0.05 to 1 M hydrochloric acid.

In Walvoort MT, Volbeda AG, Reintjens NR, van den Elst H, Plante OJ, Overkleeft HS, van der Marel GA, Codée JD, "Automated Solid-Phase Synthesis of Hyaluronan Oligosaccharides" Org. Lett., 2012, 14 (14), pp. 3776-3779, there is disclosed a method for efficient manufacture of hyaluronic acid fragments of each size of sugar units, such as the following, through a solid-phase synthesis process, and manufacture can be accomplished by such methods as well.

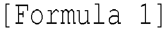

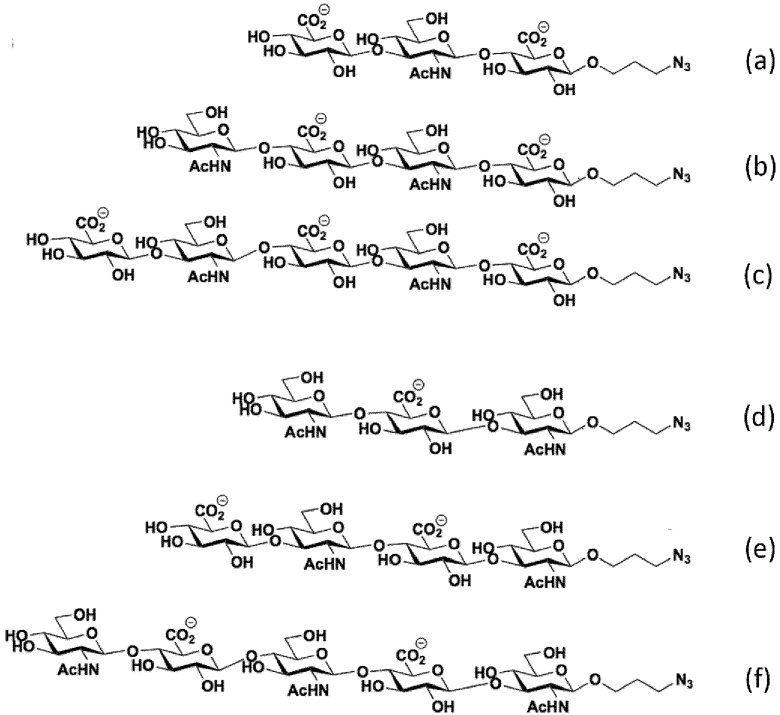

Of the above, with hyaluronic acid as the starting material, and depending on the decomposition treatment thereof, hyaluronic acid fragments having the basic structure of (a), (b), (c), (d), and (f) are substantially not obtained, or obtained at very poor yield.

### (Dosage form)

The sirtuin inducer, tissue repairing agent, hepatocyte growth factor inducer, tissue homeostasis maintenance agent, or TLR4 agonist according to the present invention contain as an active ingredient the aforedescribed hyaluronic acid fragment, a pharmaceutically acceptable salt thereof, or a pharmaceutically acceptable solvate thereof (hereinafter termed simply "hyaluronic acid fragment").

There are no particular limitations as to the amounts in which the hyaluronic acid fragment is contained, provided that the effects of the present invention are realized, but in preferred practice the amount is from 0.0001 mass% to 100 mass%, with respect to the total amount of the sirtuin inducer, tissue repairing agent, hepatocyte growth factor inducer, tissue homeostasis maintenance agent, or TLR4 agonist, more preferably from 0.01 mass% to 20 mass%, and most preferably from 0.5 mass% to 5 mass%.

The sirtuin inducer, tissue repairing agent, hepatocyte growth factor inducer, tissue homeostasis maintenance agent, or TLR4 agonist according to the present invention may be employed in the form, for example, of a pharmaceutical, functional food product, or cosmetic.

As pharmaceutical forms, there may be cited, for example, tablet form, liquid form, dry powder form, injection form, intravenous drip form, topical form, adhesive patch form, liquid form for inhaler use, suppository form, or the like. In this case, one or more pharmaceutically acceptable components or carriers may be combined during preparation. For example, excipients, fillers, disintegrants, binders, lubricants, surfactants, alcohol, water, water-soluble polymers, sweeteners, flavorings, acidulants, and the like can be added. In the case of use in topical form, for example, lubricants such as calcium stearate or emulsifiers, purified water, such as ion-exchanged water, for supplying moisture to the keratinous layer, moisturizing agents such as glycerin or PEG for moisturizing the keratinous layer, emollients (oil components for preventing evaporation of moisture), such as ester oils or vegetable oils, for improving moisturizing and application feel, solubilizing agents for solubilizing material components, buffering agents for adjusting pH, preservatives for inhibiting microbial growth and preventing spoilage, coloring agents for imparting color, discoloration inhibitors, such as UV absorbers, for preventing discoloration or change in color, styptic agents, bactericides, activators, antiphlogistics, and the like can be added.

As functional food product forms, there may be cited, for example, liquid food products, tablet food products, powdered food products, granular food products, and the like. In this case, one or more acceptable food-grade components or carriers may be combined during preparation. For example, vitamin B₁, vitamin B₂, vitamin B₆, vitamin B₁₂, folic acid, niacin, vitamin E, or other vitamins, fats and oils, thickeners, preservatives, coloring agents, antioxidants, seasoning agents, acidulants, sweeteners, and the like can be added.

As cosmetic forms, there may be cited, for example, eyedrops, gels, creams, liquid spray form, liquid mist form, foaming aerosol form, face masks of gel or sheet form, and the like. In this case, one or more cosmetically acceptable components or carriers may be combined during preparation. For example, lubricants such as calcium stearate or emulsifiers, purified water, such as ion-exchanged water, for supplying moisture to the keratinous layer, moisturizing agents such as glycerin or PEG for moisturizing the keratinous layer, emollients (oil components for preventing evaporation of moisture), such as ester oils or vegetable oils, for improving moisturizing and application feel, solubilizing agents for solubilizing material components, buffering agents for adjusting pH, preservatives for inhibiting microbial growth and preventing spoilage, coloring agents for imparting color, discoloration inhibitors, such as UV absorbers, for preventing discoloration or change in color, styptic agents, bactericides, activators, antiphlogistics, and the like can be added.

In cases in which the sirtuin inducer, tissue repairing agent, hepatocyte growth factor inducer, tissue homeostasis maintenance agent, or TLR4 agonist according to the present invention is employed in the form of a pharmaceutical, functional food product, or cosmetic, the amount in which the hyaluronic acid fragment is contained, is preferably from 0.0001 mass% to 100 mass%, more preferably from 0.01 mass% to 20 mass%, and most preferably from 0.5 mass% to 5 mass%, with respect to the total mass of the pharmaceutical, functional food product, or cosmetic.

Regarding the form of administration of the sirtuin inducer, tissue repairing agent, hepatocyte growth factor inducer, tissue homeostasis maintenance agent, or TLR4 agonist according to the present invention, these may be administered through oral ingestion, or through intravenous injection or perfusion in the form of an injection or drip. Alternatively, the agents may be applied to the skin, adsorbed through inhalation from an inhaler system, or topically applied. Administration by ocular instillation is also acceptable. In any event, there are no particular limitations as to the form of administration. The agents can be used not only in humans, but also in animals.

The dosage amount per single administration of the hyaluronic acid fragment, the dosage interval, and the like are matters to be determined individually, depending on the form of administration, the purpose of use, the specific symptoms, age, sex, and weight of the patient, and the like, and there are no particular limitations in this regard. In the case of administration through oral ingestion, an individual adult dosage of 1 mg to 7500 mg per dose may be given by way of example. In the case of application to the skin, an amount of 0.01 µg to 1000 µg/cm² per dose per unit surface area may be given by way of example. In the case of administration by ocular instillation, an applied amount of 0.01 µg to 1000 µg per dose may be given by way of example.

Because the hyaluronic acid fragment has substantially no toxicity or antigenicity, they act effectively as a sirtuin inducer, tissue repairing agent, hepatocyte growth factor inducer, tissue homeostasis maintenance agent, or TLR4 agonist with very little risk of side effects.

### (Sirtuin inducer)

Sirtuin proteins are translational products of the SIRT1 gene, and are enzymes that deacetylate histones. In cells in which sirtuin proteins are expressed, acetyl groups are removed from the histones, and the DNA is tightly wound about the histones. As a result of the DNA being tightly wound about the histones, gene expression in the region declines, thereby regulating the expression of specific genes or transcription factors. Damage to the DNA is reduced as well. Sirtuin proteins have regulating action on various cell functions, such as stress resistance, fat/glucose metabolism, neuron cell differentiation, and the like, and thus contribute to determinations as to the lifespan of cells, tissues, or individual organisms.

Therefore, the sirtuin inducer according to the present invention can promote expression of sirtuin proteins, thereby inhibiting aging in cells, tissues, individual organisms, and the like. Individual organisms may be maintained in health, prolonging life. At the cellular or physiological tissue level as well, aging can be inhibited, thus conserving the activities and lengthening the survival spans thereof.

Furthermore, it has been reported that, for example, tumor formation is inhibited through increased sirtuin expression through gene manipulation, while conversely, knocking out the sirtuin gene (SIRT1 gene) promotes cancer formation (Kabra N, Li Z, Chen L, Li B, Zhang X, Wang C, Yeatman T, Coppola D, Chen J "SirT1 is an inhibitor of proliferation and tumor formation in colon cancer" J Biol. Chem. 2009 Jul 3; 284(27): 18210-7).

It has also been reported that myocardial infarction can be treated through sirtuin induction (Samuel SM, Thirunavukkarasu M, Penumathsa SV, Paul D, Maulik N "Akt/FOXO3a/SIRT1-mediated cardioprotection by n-tyrosol against ischemic stress in rat in vivo model of myocardial infarction: switching gears toward survival and longevity. " J Agric. Food Chem. 2008 Oct 22; 56(20): 9692-8)

It has also been reported that sirtuin induction inhibits aging in the endothelial cells of blood vessels, preventing arterial sclerosis (Ota H, Eto M, Ogawa S, Iijima K, Akishita M, Ouchi Y "SIRT1/eNOS axis as a potential target against vascular senescence, dysfunction and atherosclerosis" Journal of Atherosclerosis and Thrombosis Vol. 17 (2010) No. 5 p 431-435).

Moreover, it has been reported that sirtuin induction inhibits resistin, which is a contributing factor to aortic stenosis and arterial sclerosis (Carter S, Miard S, Roy-Bellavance C, Boivin L, Li Z, Pibarot P, Mathieu P, Picard "Sirt1 inhibits resistin expression in aortic stenosis" PLoS One. 2012; 7(4):e35110).

Additionally, it has been reported that sirtuin induction inhibits NF-kappaB activation in bone cells, which contributes to destruction of bone, thus preventing osteoporosis (Shakibaei M, Buhrmann C, Mobasheri A "Resveratrol-mediated SIRT-1 interactions with p300 modulate receptor activator of NF-kappaB ligand (RANKL) activation of NF-kappaB signaling and inhibit osteoclastogenesis in bone-derived cells" J Biol Chem. 2011 Apr 1; 286(13): 11492-505).

Additionally, it has been reported that sirtuin induction inhibits NF-kappaB, a factor exacerbating inflammation, whereby the symptoms of inflammatory colitis can be reduced (Singh UP, Singh NP, Singh B, Hofseth LJ, Price RL, Nagarkatti M, Nagarkatti PS "Resveratrol (trans-3,5,4'-trihydroxystilbene) induces silent mating type information regulation-1 and down-regulates nuclear transcription factor-kappaB activation to abrogate dextran sulfate sodium-induced colitis" J Pharmacol. Exp. Ther. 2010 Mar; 332(3): 829-39).

Moreover, it has been reported that sirtuin proteins inhibit the production of amyloid β, a factor contributing to dementia (Alzheimer's disease) (Donmez G, Wang D, Cohen DE, Guarente L "SIRT1 suppresses beta-amyloid production by activating the alpha-secretase gene ADAM10" Cell 2010 Jul 23; 142(2): 320-32).

Sirtuin induction was reportedly found to have therapeutic effect in various animal models of Alzheimer's disease (Donmez G "The Effects of SIRT1 on Alzheimer's Disease Models" Int. J. Alzheimers Dis. 2012; 2012: 509529).

It has also been reported that sirtuin proteins are involved in the inhibiting action of encephalopathy by nicotinamide phosphoribosyltransferase in a model of cerebral apoplexy (ischemic stroke) (Wang P, Xu TY, Guan YF, Tian WW, Viollet B, Rui YC, Zhai QW, Su DF, Miao CY "Nicotinamide phosphoribosyltransferase protects against ischemic stroke through SIRT1-dependent adenosine monophosphate-activated kinase pathway" Ann. Neurol. 2011 Feb; 69(2): 360-74).

It has also been reported that in a sirtuin protein enhanced-expression model (through gene manipulation), disorders stemming from metabolic syndrome, and associated cancers, are inhibited, as compared with normal animals (Herranz D, Muñoz-Martin M, Cañamero M, Mulero F, Martinez-Pastor B, Fernandez-Capetillo O, Serrano M "Sirt1 improves healthy ageing and protects from metabolic syndrome-associated cancer" Nat. Commun. 2010 Apr 12; 1:3).

It has also been reported that tumor formation is inhibited through enhanced expression of sirtuin proteins through gene manipulation, and conversely that weakened sirtuin protein expression promotes tumor formation (Kabra N, Li Z, Chen L, Li B, Zhang X, Wang C, Yeatman T, Coppola D, Chen J "SirT1 is an inhibitor of proliferation and tumor formation in colon cancer" J. Biol. Chem. 2009 Jul 3; 284(27): 18210-7).

Moreover, in relation to inflammation or thrombosis of the lungs caused by airborne particulate matter, sirtuin proteins have been reported as an inhibitory factor of the action thereof (Wu Z, Liu MC, Liang M, Fu J "Sirt1 protects against thrombomodulin down-regulation and lung coagulation after particulate matter exposure" Blood 2012 Mar 8; 119(10): 2422-9).

It has also been reported that cigarette smoking induces autophagy in lung cells through action dependent on sirtuin proteins, which is a cause of COPD (chronic obstructive pulmonary disease), and that such autophagy is inhibited through sirtuin induction (Hwang JW, Chung S, Sundar IK, Yao H, Arunachalam G, McBurney MW, Rahman I "Cigarette smoke-induced autophagy is regulated by SIRT1-PARP-1-dependent mechanism: implication in pathogenesis of COPD" Arch. Biochem. Biophys. 2010 Aug 15; 500(2): 203-9).

It has also been reported that administration of a sirtuin activator in a model of ischemia/reperfusion disorders in the kidney inhibits cell death in the kidney, reducing kidney disorders (Fan H, Yang HC, You L, Wang YY, He WJ, Hao CM "The histone deacetylase, SIRT1, contributes to the resistance of young mice to ischemia/reperfusion-induced acute kidney injury" Kidney Int. 2013 Jan 9).

Sirtuin induction has also been reported to reduce cisplatin-induced toxicity to the tubules of the kidney and NF-kappaB activation (Jung YJ, Lee JE, Lee AS, Kang KP, Lee S, Park SK, Lee SY, Han MK, Kim DH, Kim W "SIRT1 overexpression decreases cisplatin-induced acetylation of NF-κB p65 subunit and cytotoxicity in renal proximal tubule cells" Biochem. Biophys. Res. Commun. 2012 Mar 9; 419(2): 206-10).

Sirtuin proteins have been reported to be involved in the mechanism by which insulin resistance is generated (Chen YR, Lai YL, Lin SD, Li XT, Fu YC, Xu WC "SIRT1 interacts with metabolic transcriptional factors in the pancreas of insulin-resistant and calorie-restricted rats" Mol. Biol. Rep. 2013 Jan 6).

Sirtuin proteins have also been reported to be involved in the mechanism of action of the antihypertensive Telmisartan in ameliorating insulin sensitivity (Shiota A, Shimabukuro M, Fukuda D, Soeki T, Sato H, Uematsu E, Hirata Y, Kurobe H, Maeda N, Sakaue H, Masuzaki H, Shimomura I, Sata M "Telmisartan ameliorates insulin sensitivity by activating the AMPK/SIRT1 pathway in skeletal muscle of obese db/db mice" Cardiovasc, Diabetol. 2012 Nov 8; 11:139).

Sirtuin proteins have also been reported to inhibit TNFα-induced inflammation in human chondrocytes (Moon MH, Jeong JK, Lee YJ, Seol JW, Jackson CJ, Park SY "SIRT1, a class III histone deacetylase, regulates TNF-α-induced inflammation in human chondrocytes" Osteoarthritis Cartilage. 2013 Mar; 21(3): 470-80).

Sirtuin protein overexpression in human chondrocytes is reported to inhibit the expression of osteoarthritis factors even when treated with IL-1beta (Matsushita T, Sasaki H, Takayama K, Ishida K, Matsumoto T, Kubo S, Matsuzaki T, Nishida K, Kurosaka M, Kuroda R"The overexpression of SIRT1 inhibited osteoarthritic gene expression changes induced by interleukin-1β in human chondrocytes. " J Orthop. Res. 2012 Nov 9.

Sirtuin proteins have also been reported to be involved in the action of aggravating factors of rheumatism (Kok SH, Lin LD, Hou KL, Hong CY, Chang CC, Hsiao M, Wang JH, Lai EH, Lin SK "Simvastatin inhibits Cyr61 expression in rheumatoid arthritis synovial fibroblasts through the regulation of SIRT1/FoxO3a signaling" Arthritis Rheum. 2012 Dec 12).

A sirtuin activator has been reported to show disease-alleviating effect in a progeria animal model (Liu B, Ghosh S, Yang X, Zheng H, Liu X, Wang Z, Jin G, Zheng B, Kennedy BK, Suh Y, Kaeberlein M, Tryggvason K, Zhou Z "Resveratrol rescues SIRT1-dependent adult stem cell decline and alleviates progeroid features in laminopathy-based progeria" Cell Metab. 2012 Dec 5; 16(6): 738-50).

Radiation injury are caused by cell death through translocation of GAPDH from cytoplasm to nucleus, and sirtuin proteins have been reported to inhibit this translocation of GAPDH to nucleus, inhibiting cell death (Joo HY, Woo SR, Shen YN, Yun MY, Shin HJ, Park ER, Kim SH, Park JE, Ju YJ, Hong SH, Hwang SG, Cho MH, Kim J, Lee KH "SIRT1 interacts with and protects glyceraldehyde-3-phosphate dehydrogenase (GAPDH) from nuclear translocation: implications for cell survival after irradiation" Biochem. Biophys. Res. Commun. 2012 Aug 10; 424(4): 681-6).

Sirtuin proteins are also reported not only as strengthening muscle fibers, but also as acting to protect the muscles from various diseases (Tonkin J, Villarroya F, Puri PL, Vinciguerra M "SIRT1 signaling as potential modulator of skeletal muscle diseases" Curr. Opin. Pharmacol. 2012 Jun; 12 (3) : 372-6).

Enhanced expression of sirtuin proteins promotes dendrite formation in hippocampal neuron cells, while conversely the action of a sirtuin inhibitor induces dendrites to atrophy; further, enhanced expression of sirtuin proteins also inhibits dendritic atrophy due to treatment with amyloid β, which means that sirtuin proteins are responsible for development and maintenance of neural tissue in the hippocampus and the like (Codocedo JF, Allard C, Godoy JA, Varela-Nallar L, Inestrosa NC "SIRT1 regulates dendritic development in hippocampal neurons" PLoS One. 2012; 7(10): e47073).

Sirtuin proteins are also reported to inhibit angiotensin II, a factor inducing hypertension (Li L, Gao P, Zhang H, Chen H, Zheng W, Lv X, Xu T, Wei Y, Liu D, Liang C "SIRT1 inhibits angiotensin II-induced vascular smooth muscle cell hypertrophy" Acta Biochim. Biophys. Sin. (Shanghai). 2011 Feb; 43(2): 103-9).

Sirtuin induction is reported to inhibit insulin resistance and obesity (Li H, Xu M, Lee J, He C, Xie Z "Leucine supplementation increases SIRT1 expression and prevents mitochondrial dysfunction and metabolic disorders in high-fat diet-induced obese mice" Am. J. Physiol. Endocrinol. Metab. 2012 Nov 15; 303(10): E1234-44.)

Accordingly, the sirtuin inducer according to the present invention is favorable for use particularly in treatment and/or prevention of heart disease, arterial sclerosis, osteoporosis, inflammatory bowel disease, dementia, apoplexy, metabolic syndrome, cancer, pulmonary disease, renal disease, diabetes, osteoarthritis, rheumatism, progeria, radiation injury, muscle disorder, brain development disorder, neurological illness, hypertension, or obesity, or to inhibit aging.

### (Tissue repairing agent)

The tissue repairing agent according to the present invention promotes repair of tissue in wound regions or missing regions in biological tissue. More specifically, by promoting cell growth, or inducing cell differentiation, in wound regions or missing regions in biological tissue, repair of tissue in wound regions or missing regions in biological tissue can be promoted.

For example, the agent is effective in treatment of various injuries such as decubitus (bedsores), neutrophilic ulcers, burn injuries, painless ulcers, posttraumatic ulcers, ulcers due to venous hematoma of veins and post-venous inflammation, skin lesions, and cutaneous affections stemming from skin grafts and herpes simplex. The agent is favorable for use in repairing, for example, the cornea, dermis, epithelial tissue, neurological tissue, or cardiac muscle tissue.

### (Hepatocyte growth factor inducer)

Hepatocyte growth factor (HGF) is a protein having a heterodimer structure in which heavy chains of molecular weight of about 60,000 and light chains of molecular weight of about 35,000 are bonded through disulfide bonds. Hepatocyte growth factor, once synthesized in the body, upon elimination of of N-terminal signal peptides and secretion from the cell, is cleaved into two chains by HGF activator or urokinase (u-PA), and becomes the active form.

Hepatocyte growth factor not only promotes propagation of liver cells, but also acts primarily on epithelial cells, those derived from major organs, and on vascular endothelial cells, hematopoietic lineage cells, and neural cells, activating and promoting the growth of various cells. The agent is also involved in activation of skin and the like. For example, hepatocyte growth factor acts on epithelial cells such as keratinocytes, and on hair matrix or follicle cells and the vascular endothelial cells just below, activating the cells. Furthermore, hepatocyte growth factor also has cell motility-enhancing action, morphogenesis-inducing action such as lumen formation or the like, anti-aptosis action, angiogenetic action, an immunological response regulating action. In this way, hepatocyte growth factor plays an important role within the body.

Therefore, the hepatocyte growth factor inducer according to the present invention can promote expression of the hepatocyte growth factor, thereby activating the cells of tissues and various organs including the liver, heart, stomach blood vessels, and skin, or maintaining activity thereof, and providing protection thereof from disease factors such as inflammation, bacteria, or viruses, or from injuries over time or external injuries. The agent is favorable for use in treatment and/or prevention of liver disorders, or for organ preservation.

Another aspect of the hepatocyte growth factor inducer according to the present invention is the HGF morphogenic inducing action and angiogenic action. For example, the agent holds promise in terms of effective healing of wounds of blood vessels, skin, stomach, and the like. Moreover, the agent holds promise in terms of significant effectiveness in preventing inflammation of various kinds, such as hepatitis and other liver disorders, nephritis and other kidney disorders, pneumonia and other lung disorders, and the like.

Yet another aspect of the hepatocyte growth factor inducer according to the present invention relates to the use thereof in cosmetic form. The cosmetic according to the present invention has the hyaluronic acid fragment, and thereby has the effect of promoting expression of hepatocyte growth factor. Therefore, the agent can suppress inflammation of the skin, for example. By activating cells on biological surfaces, the agent can promote cell growth on biological surfaces and exhibit anti-aging effect. For example, Japanese Laid-Open Patent Application 5-213733 discloses a skin cosmetic having hepatocyte growth factor (HGF) as an active ingredient, which can improve sunburn-induced chapping of the skin, and which has the effect of improving small wrinkles of the skin to make the skin more beautiful. The cosmetic according to the present invention, like the cosmetic disclosed in this publication, functions as a skin cosmetic for improving sunburn-induced chapping of the skin, and improving small wrinkles of the skin to make the skin more beautiful.

### (Tissue homeostasis maintenance agent)

Tissue aging is reportedly due to depletion of tissue stem cells (Nishimura EK, Granter SR, Fisher DE "Mechanisms of hair graying: incomplete melanocyte stem cell maintenance in the niche" Science, 2005 Feb 4; 307(5710): 720-4). That is, tissue aging arises due to a failure of neoplastic propagation by cells in tissue, or to dysfunction of constituent cells. Consequently, prevention of tissue aging requires: (1) maintenance and growth of tissue stem cells; (2) induction of matured/differentiated cells which are functional cells; and (3) full functionality on the part of matured/differentiated cells.

Examples of human keratinocytes and the human neurosphere in the examples discussed below demonstrate that the hyaluronic acid fragment both maintain and induce differentiation of tissue stem cells.

Additionally, the hepatocyte growth factor inducer according to the present invention, particularly through the ability to maintain stemness and induce differentiation in cells of tissue, inhibits aging at the biological tissue level, and thus can maintain activity and extend the duration of survival thereof.

### (TLR4 agonist)

Through the examples discussed below, it was demonstrated that the hyaluronic acid fragment promotes expression of sirtuin proteins, this induction of expression taking place via the action of TLR4 (toll-like receptor 4). Additionally, the hyaluronic acid fragment was demonstrated to inhibit increase in the expression level of NF-kappaB triggered by TNF-α treatment, this inhibition also taking place via the action of TLR4. Furthermore, the hyaluronic acid fragment was demonstrated to have the effect of acting to inhibit cell aging due to various factors, this inhibition also taking place via the action of TLR4. Furthermore, the hyaluronic acid fragment was demonstrated to have the effect of acting to alleviate disorders of tissue after preservation in tissue preservation solution, this alleviation also taking place via the action of TLR4. Furthermore, the hyaluronic acid fragment was demonstrated to induce cell differentiation, this differentiation also taking place via the action of TLR4.

TLR4 (toll-like receptor 4) has been discovered to be a receptor that recognizes lipopolysaccharides (LPS) of Gram-negative bacteria, and is known to be a receptor that, for example, activates NF-kappaB, which causes inflammation. Apart from activation of NF-kappaB, as shown in the following documents, TLR4 is known to be expressed in various organs/cells such as the cornea, skin, liver, neurological tissue, kidneys, lungs, heart, skeletal muscles, pancreas, blood vessels, and the like, and to be involved in various biological functions. Therefore, TLR4 can be applied in the treatment and prevention of the various diseases indicated below.

### •Cornea (corneal epithelium, keratocytes)

Corneal disease: dry eye, corneal epithelium disorders, separation of the corneal epithelium, destruction of corneal tissue by inflammation, and the like.

Johnson AC, Heinzel FP, Diaconu E, Sun Y, Hise AG, Golenbock D, Lass JH, Pearlman E "Activation of toll-like receptor (TLR)2, TLR4, and TLR9 in the mammalian cornea induces MyD88-dependent corneal inflammation" Invest. Ophthalmol. Vis. Sci. 2005 Feb; 46(2): 589-95.

### •Skin (keratinocytes, Langhans cells, fibroblast cells)

Skin disease: decubitus, cutaneous ulcers, destruction of skin tissue by infection/inflammation and the like
1. Tuon FF, Fernandes ER, Duarte MI, Amato VS "The expression of TLR2, TLR4 and TLR9 in the epidermis of patients with cutaneous leishmaniasis" J. Dermatol. Sci. 2010 Jul; 59(1): 55-7
2. Yuan ZQ, Bennett L, Campo MS, Nasir L "Bovine papillomavirus type 1 E2 and E7 proteins down-regulate Toll Like Receptor 4 (TLR4) expression in equine fibroblasts" Virus Res. 2010 Apr; 149(1): 124-7
3. Wang X, Bi Z, Wang Y, Wang Y "Increased MAPK and NF-κB expression of Langerhans cells is dependent on TLR2 and TLR4, and increased IRF-3 expression is partially dependent on TLR4 following UV exposure" Mol. Med. Report. 2011 May-Jun; 4(3): 541-6

### •Liver (hepatic parenchymal cells, Kupffer's cells)

Acute hepatitis, chronic hepatitis, liver transplant disorders, and the like
1. Sahin H, Borkham-Kamphorst E, do O NT, Berres ML, Kaldenbach M, Schmitz P, Weiskirchen R, Liedtke C, Streetz KL, Maedler K, Trautwein C, Wasmuth HE "Proapoptotic effects of the chemokine, CXCL 10 are mediated by the noncognate receptor TLR4 in hepatocytes" Hepatology. 2012 Sep 19, doi: 10.1002/hep.26069
2. Bai T, Lian LH, Wu YL, Wan Y, Nan JX "Thymoquinone attenuates liver fibrosis via PI3K and TLR4 signaling pathways in activated hepatic stellate cells" Int. Immunopharmacol. 2013 Jan 12; 15(2): 275-281

### •Neurological tissue (neuron cells, microglia cells)

Neurological disease: dementia, neurodevelopmental disorders, neurological tissue degeneration, inhibited neural differentiation, and the like
1. Capiralla H, Vingtdeux V, Zhao H, Sankowski R, Al-Abed Y, Davies P, Marambaud P "Resveratrol mitigates lipopolysaccharide- and Aβ-mediated microglial inflammation by inhibiting the TLR4/NF-κB/STAT signaling cascade" J. Neurochem. 2012 Feb; 120(3): 461-72
2. Okun E, Barak B, Saada-Madar R, Rothman SM, Griffioen KJ, Roberts N, Castro K, Mughal MR, Pita MA, Stranahan AM, Arumugam TV, Mattson MP "Evidence for a developmental role for TLR4 in learning and memory" PLoS One. 2012; 7(10): e47522
3. Hutchinson MR, Zhang Y, Brown K, Coats BD, Shridhar M, Sholar PW, Patel SJ, Crysdale NY, Harrison JA, Maier SF, Rice KC, Watkins LR "Non-stereoselective reversal of neuropathic pain by naloxone and naltrexone: involvement of toll-like receptor 4 (TLR4)" Eur. J. Neurosci. 2008 Jul; 28(1): 20-9

### •Kidney (mesangial cells, renal epithelial cells)

Nephritis, nephropathy, and the like
1. Lee IT, Shih RH, Lin CC, Chen JT, Yang CM "Role of TLR4/NADPH oxidase/ROS-activated p38 MAPK in VCAM-1 expression induced by lipopolysaccharide in human renal mesangial cells" Cell Commun. Signal. 2012 Nov 15; 10(1): 33
2. Good DW, George T, Watts BA 3rd "Lipopolysaccharide directly alters renal tubule transport through distinct TLR4-dependent pathways in basolateral and apical membranes" Am. J. Physiol. Renal Physiol. 2009 Oct; 297(4): F866-74

### •Lungs (epithelial cells, stromal cells, smooth muscle)

Pneumonia, COPD, ischemia/reperfusion disorders, and the like
1. Perros F, Lambrecht BN, Hammad H "TLR4 signalling in pulmonary stromal cells is critical for inflammation and immunity in the airways" Respir. Res. 2011 Sep 24; 12: 125
2. Su HY, Mo BW, Wei JH, Huang JW, Wang CM, Zeng JR, Xu Q, Lin Y "Effect of TLR4 on the migration of asthmatic airway smooth muscle cells induced by airway epithelial cells" Zhongguo Ying Yong Sheng Li Xue Za Zhi, 2012 Mar; 28(2): 103-6

### •Heart (cardiac muscle)

### Cardiac infarction, angina, and the like

Frantz S, Kobzik L, Kim YD, Fukazawa R, Medzhitov R, Lee RT, Kelly RA "Toll4 (TLR4) expression in cardiac myocytes in normal and failing myocardium" J. Clin. Invest. 1999 Aug; 104 (3) : 271-80

### •Skeletal muscles (skeletal muscle cells)

Muscular atrophy, polymyositis, myasthenia gravis, muscular dystrophy, and the like

Zbinden-Foncea H, Raymackers JM, Deldicque L, Renard P, Francaux M "TLR2 and TLR4 activate p38 MAPK and JNK during endurance exercise in skeletal muscle" Med. Sci. Sports Exerc. 2012 Aug; 44(8): 1463-72

### •Pancreas

### Pancreatitis

1. Akbarshahi H, Axelsson JB, Said K, Malmström A, Fischer H, Andersson R "TLR4 dependent heparan sulphate-induced pancreatic inflammatory response is IRF3-mediated" J. Transl. Med. 2011 Dec 21; 9: 219
2. Awla D, Abdulla A, Regnér S, Thorlacius H "TLR4 but not TLR2 regulates inflammation and tissue damage in acute pancreatitis induced by retrograde infusion of taurocholate: Inflamm. Res. 2011 Dec; 60(12): 1093-8

### •Arteries (smooth muscle)

### Arterial sclerosis and the like

Li H, Xu H, Sun B "Lipopolysaccharide regulates MMP-9 expression through TLR4/NF-κB signaling in human arterial smooth muscle cells" Mol Med Report. 2012 Oct; 6(4):774-8

Therefore, through the action of TLR4, the TLR4 agonist according to the present invention can treat and/or prevent various diseases. For example, the agent can be used appropriately to treat and/or prevent corneal diseases such as dry eye, corneal epithelium disorders, separation of the corneal epithelium, destruction of corneal tissue by inflammation, and the like; skin diseases such as decubitus, cutaneous ulcers, destruction of skin tissue by infection/inflammation and the like; liver disease such as acute hepatitis, chronic hepatitis, liver transplant disorders, and the like; neurological disease such as dementia, neurodevelopmental disorders, neurological tissue degeneration, inhibited neural differentiation, and the like; kidney disease such as nephritis, nephropathy, and the like; lung disease such as pneumonia, COPD, ischemia/reperfusion disorders, and the like; heart disease such as cardiac infarction, angina, and the like; skeletal muscle disease such as muscular atrophy, polymyositis, myasthenia gravis, muscular dystrophy, and the like; pancreatic disease such as pancreatitis and the like; and blood vessel disease such as arterial sclerosis and the like.

### [Examples]

The present invention is described in more specific terms below through examples, but these examples are not limiting of the scope of the present invention.

The hyaluronic acid fragments used in the following examples are shown below.

### (1) 4mer

### "HYA-OLIGO4EF-5" (trade name of Hyalose LLC)

A hyaluronic acid fragment of four-unit size having the structure GlcA(-GlcNAc-GlcA)₁-GlcNAc, obtained through enzymatic decomposition and purification of hyaluronic acid.

### (2) 6mer

### "HYA-OLIGO6EF-1" (trade name of Hyalose LLC)

A hyaluronic acid fragment of six-unit size having the structure GlcA(-GlcNAc-GlcA)₂-GlcNAc, obtained through enzymatic decomposition and purification of hyaluronic acid.

### (3) 8mer

### "HYA-OLIGO8EF-1" (trade name of Hyalose LLC)

A hyaluronic acid fragment of eight-unit size having the structure GlcA(-GlcNAc-GlcA)₃-GlcNAc, obtained through enzymatic decomposition and purification of hyaluronic acid.

### (4) 10mer

### "HYA-OLIGO10EF-1" (trade name of Hyalose LLC)

A hyaluronic acid fragment of eight-unit size having the structure GlcA(-GlcNAc-GlcA)₄-GlcNAc, obtained through enzymatic decomposition and purification of hyaluronic acid.

### (5) L3mer

A hyaluronic acid fragment having the following structure (a). 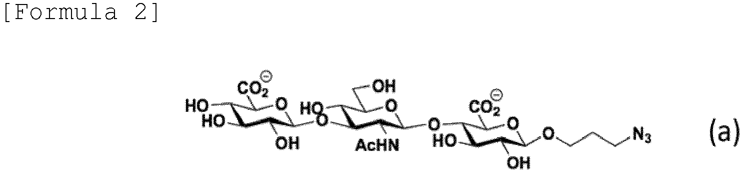 This is a hyaluronic acid fragment of three-unit size chemically synthesized and purified by the solid-phase synthesis process (Walvoort MT, Volbeda AG, Reintjens NR, van den Elst H, Plante OJ, Overkleeft HS, van der Marel GA, Codée JD, "Automated Solid-Phase Synthesis of Hyaluronan Oligosaccharides" Org. Lett., 2012, 14 (14), pp. 3776-3779).

### (6) L4mer

A hyaluronic acid fragment having the following structure (e). 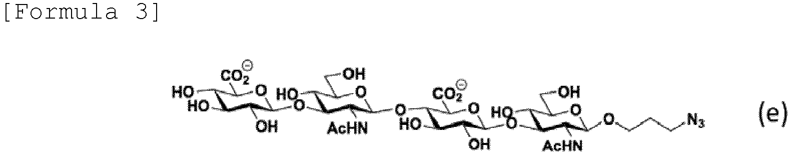 This is a hyaluronic acid fragment of four-unit size chemically synthesized and purified by the solid-phase synthesis process (Walvoort MT, Volbeda AG, Reintjens NR, van den Elst H, Plante OJ, Overkleeft HS, van der Marel GA, Codée JD, "Automated Solid-Phase Synthesis of Hyaluronan Oligosaccharides" Org. Lett., 2012, 14 (14), pp. 3776-3779).

### (Test Example 1) (Sirtuin induction in human epidermal keratinocytes Part 1)

Human epidermal keratinocytes (normal human keratinocytes from an Asian female (age 27)) were cultured and prepared. Following culture of the human keratinocytes in a prolonged culture growth medium (EpiLife-KG2) at 37°C under 5% CO₂, the cells were recovered by trypsin treatment. The cells were then inoculated into a 96-well plate (100 µL per well) and cultured overnight. Following completion of culture, the culture broth from each well was transferred to a culture medium (keratinocyte SFM made by Gibco) to which no hyaluronic acid fragments were added, or to the same culture medium to which a hyaluronic acid fragment (4mer) was added to a final concentration of 0.01 ng/ml, 0.1 ng/ml, 1.0 ng/ml, or 10 ng/ml, and cultured for 24 hours.

After 24 hours had passed, the cells in each well were stained with the anti human SIRT1 antibody. The anti human SIRT1 antibody is an antibody that recognizes and stains human sirtuin proteins. The stained cells were observed under a microscope (made by Nikon), taking photographs of cell morphology and stained images of cells.

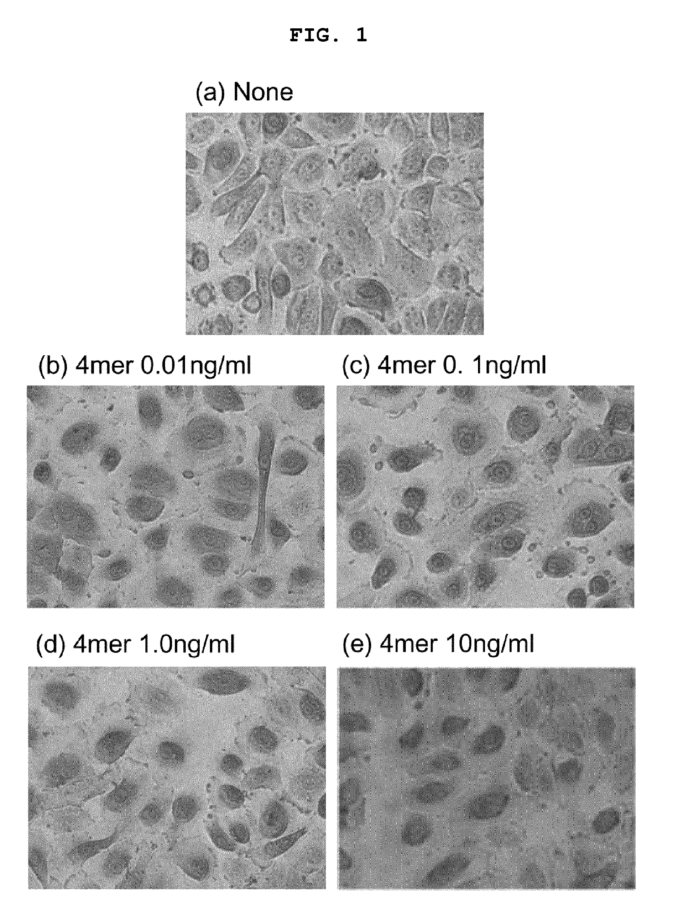

Photographs of stained images are shown in FIG. 1. FIG. 1 (a) shows human epidermal keratinocytes cultured in a culture broth to which no hyaluronic acid fragments were added. FIG. 1 (b) shows human epidermal keratinocytes cultured in a culture broth 0.01 ng/ml of hyaluronic acid fragment (4mer). FIG. 1 (c) shows human epidermal keratinocytes cultured in a culture broth 0.1 ng/ml of hyaluronic acid fragment (4mer). FIG. 1 (d) shows human epidermal keratinocytes cultured in a culture broth 1.0 ng/ml of hyaluronic acid fragment (4mer). FIG. 1 (e) shows human epidermal keratinocytes cultured in a culture broth 10 ng/ml of hyaluronic acid fragment (4mer).

As shown in FIG. 1 (a), among the human epidermal keratinocytes cultured in culture broth to which no hyaluronic acid fragments were added, cells stained by the anti human SIRT1 antibody were few in number, and of the stained cells, the cells were lightly stained overall.

On the other hand, as shown in FIG. 1 (b) to (e), among the human epidermal keratinocytes cultured in culture broth containing the hyaluronic acid fragment, cells stained by the anti human SIRT1 antibody increased in number, and the cell nuclei in particular were darkly stained. From these results, it may be appreciated that the expression of sirtuin proteins in human epidermal keratinocytes is promoted by the hyaluronic acid fragment.

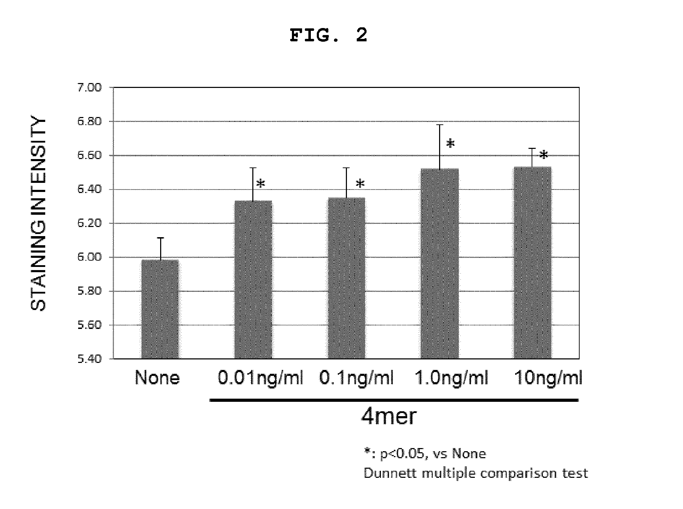

Results of analysis of the aforedescribed stained images by image analysis software ("Image J" freeware) are shown in Table 1. FIG. 2 shows a graph of the results from Table 1. The staining intensity in the results shows the extent of staining by the anti human SIRT1 antibody, with higher values being considered to indicate a higher sirtuin protein expression levels.

**[Table 1]**

| | Average staining intensity | Standard deviation |
|---|---|---|
| Negative Control (None) | 5.99 | 0.13 |
| 4mer 0.01ng/ml | 6.33 | 0.20 |
| 4mer 0.1ng/ml | 6.35 | 0.18 |
| 4mer 1.0ng/ml | 6.52 | 0.26 |
| 4mer 10ng/ml | 6.54 | 0.11 |

As shown in Table 1 or FIG. 2, among human epidermal keratinocytes cultured in culture broth containing the hyaluronic acid fragment at concentrations of 0.01 ng/ml to 10 ng/ml, the sirtuin protein expression levels increased significantly, as compared with the expression level in human epidermal keratinocytes cultured in culture broth containing no hyaluronic acid fragments.

### (Test Example 2) (Sirtuin induction in human epidermal keratinocytes Part 2)

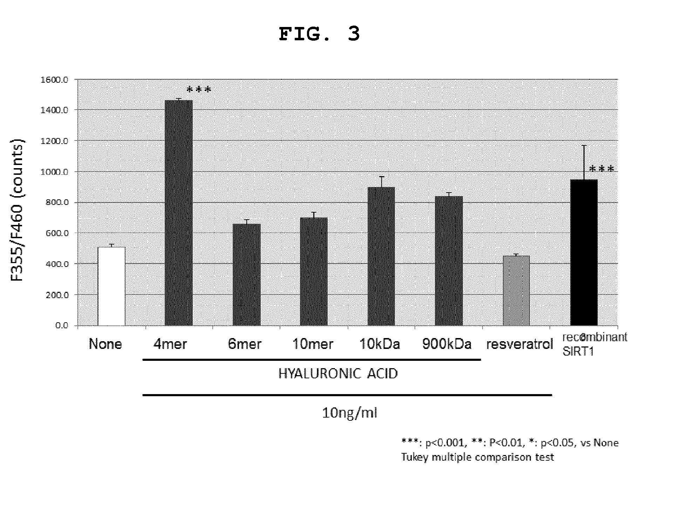

Human epidermal keratinocytes were treated in the same manner as in Test Example 1, except that the final concentration of the hyaluronic acid fragment was 10 ng/ml, the size thereof being four sugar units (4mer), six sugar units (6mer), or 10 sugar units (10mer), while measuring the sirtuin enzymatic activity (sirt1 enzymatic activity) in the cell solution thereof. For purposes of comparison, 10 kDa or 900 kDa hyaluronic acid, and resveratrol, a known sirtuin activator, were likewise tested at 10 ng/ml final concentration. A human sirtuin recombinant protein (recombinant human sirt1) produced through genetic recombination techniques was used as a substance for verification of the measurement system. Sirtuin enzymatic activity in the cell crude extract was measured with the CycLex SIRT1/Sir2 Deacetylase Fluorometric Assay Kit (trade name of CycLex), following the protocol of the kit. Results are shown in FIG. 3.

As shown in FIG. 3, with four-unit size hyaluronic acid fragment (4mer), measured values were approximately triple as compared with those of the negative control. No change was observed in the case of addition of an equal amount of resveratrol, a known sirtuin activator, and it may therefore be appreciated that four-unit size hyaluronic acid fragment (4mer) has marked effect in inducing sirtuin enzymatic activity.

### (Test Example 3) (Sirtuin induction in human epidermal keratinocytes Part 3)

A test of sirtuin induction in human epidermal keratinocytes was conducted in the same manner as in Test Example 1.

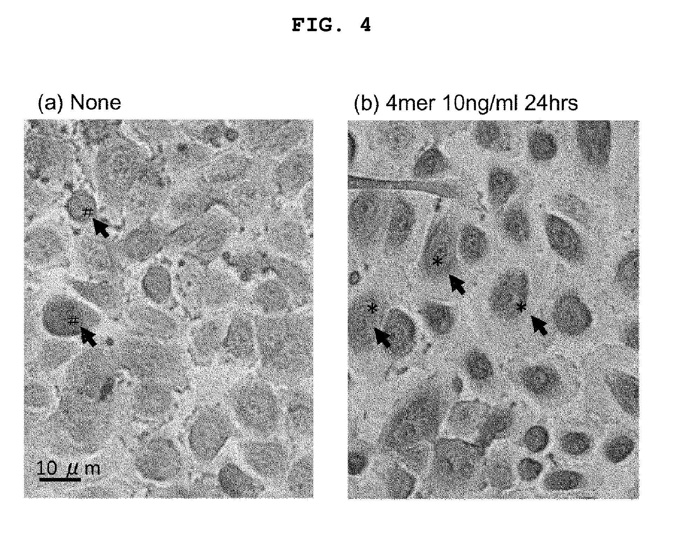

FIG. 4 shows photographs of stained images of cells stained with the anti human SIRT1 antibody. FIG. 4 (a) shows human epidermal keratinocytes cultured in culture broth to which no hyaluronic acid fragments were added. FIG. 4 (b) shows human epidermal keratinocytes cultured in culture broth containing 10 ng/ml of hyaluronic acid fragment (4mer).

As shown in FIG. 4, in the negative control of FIG. 4 (a), sirtuin protein expression was observed primarily in small, round cells (cells are indicated by arrows in the drawing). Relatively small immature cells were predominant among the cell bodies. On the other hand, as shown in FIG. 4 (b), through treatment with the hyaluronic acid fragment, cells of larger size, i.e., mature cells, appeared predominantly, and sirtuin protein expression was observed in these cells (cells are indicated by arrows in the drawing).

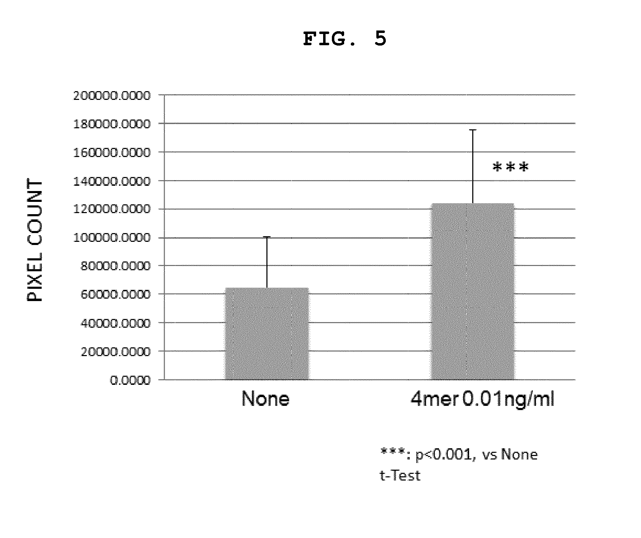

The change in cell size was analyzed using image analysis software ("Image J"). Results are shown in Table 2. The results in Table 2 are graphed in FIG. 5. The pixel count in the results indicates surface area on the image of a single cell, with higher values considered to indicate larger cells.

**[Table 2]**

| | Average pixel count | Standard deviation |
|---|---|---|
| Negative Control (None) | 64663.2667 | 35496.7006 |
| 4mer 0.01ng/ml | 124515.9333 | 51093.044 |

As shown in Table 2 and FIG. 5, larger cells appeared upon treatment with the hyaluronic acid fragment for 24 hours. Separately, once it was confirmed through staining with Alcian blue that extracellular matrix secretion was normal, cell viability was concluded to be undiminished, and this morphological change was attributed to differentiation, rather than to hypertrophy which is a sign of cell aging. While results obtained with treatment with hyaluronic acid fragment (4mer) at a final concentration of 0.01 ng/ml are shown here, similar morphological changes were observed in treatment using hyaluronic acid fragment of four-unit size (4mer), six-unit size (6mer), and eight-unit size (8mer), at final concentrations of 0.01 ng/ml, 0.1 ng/ml, 1.0 ng/ml, and 10 ng/ml.

### (Test Example 4) (Sirtuin induction in human epidermal keratinocytes Part 4)

A test of sirtuin induction in human epidermal keratinocytes was conducted in the same manner as in Test Example 1.

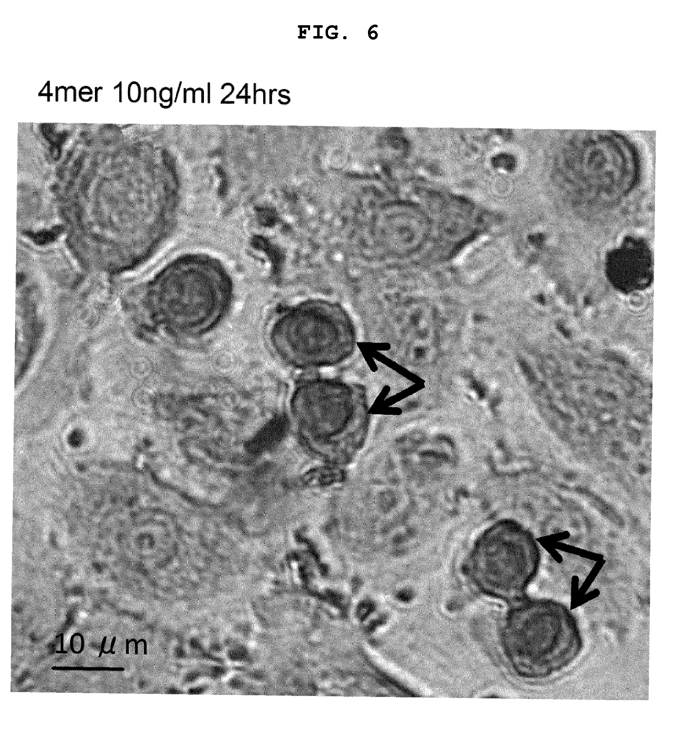

FIG. 6 shows a photograph of a stained image of cells stained with the anti human SIRT1 antibody. FIG. 6 shows human epidermal keratinocytes cultured for 24 hours in a culture broth containing 10 ng/ml of hyaluronic acid fragment (4mer).

The dividing cells indicated by arrows in FIG. 6 were small compared with others, and sirtuin protein expression in such cells was high. Ordinarily, tissue stem cells have higher proliferative potential (division potential) than do mature cells, and in terms of morphology, are known to be smaller in comparison with mature cells. Sirtuin protein expression in stem cells is reported to be high. Therefore, it may be appreciated that the dividing cells with high sirtuin protein expression shown by the arrows in FIG. 6 are stem cells of human epidermal keratinocytes.

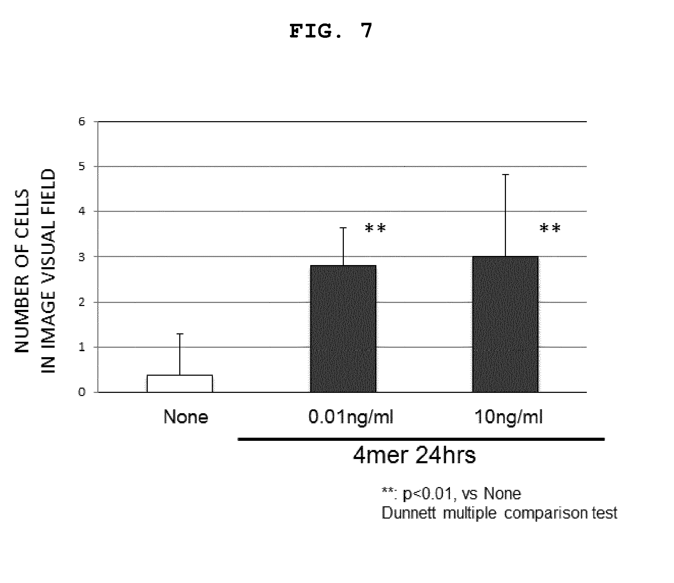

FIG. 7 shows the results of compilation of cell counts for dividing cells which, like the cells shown by the arrows in FIG. 6, are of smaller morphology than other cells, and exhibit high sirtuin protein expression. The results are shown as average count within the observed visual field.

As shown in FIG. 7, in cases of either treatment with hyaluronic acid fragment (4mer) at a final concentration of 0.01 ng/ml, or treatment with hyaluronic acid fragment (4mer) at a final concentration of 10 ng/ml, sirtuin protein expression in stem cells of human epidermal keratinocytes was markedly promoted, as compared with a negative control.

### (Test Example 5) (Sirtuin induction in human epidermal keratinocytes Part 5)

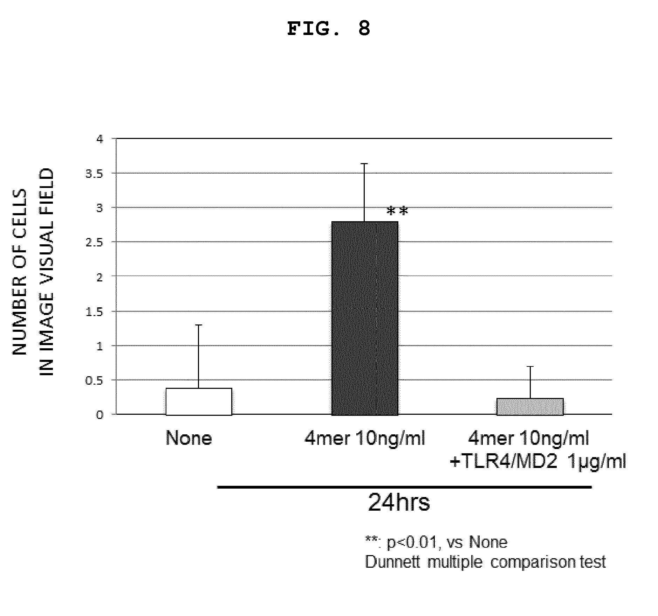

In the same manner as in Test Example 4, dividing cells of smaller morphology than other cells, and exhibiting high sirtuin protein expression, were observed. In the meantime, similar observations were made in relation to human epidermal keratinocytes cultured in a culture broth to which 10 ng/ml of hyaluronic acid fragment (4mer) and 1 µg/ml of TLR4/MD2 (TLR4 (toll-like receptor 4) protein produced by genetic recombination techniques, made by R&D Systems Inc.) were added. The results are shown in FIG. 8.

As shown in FIG. 8, sirtuin induction by hyaluronic acid fragment (4mer) in human epidermal keratinocyte stem cells was completely inhibited through the addition of TLR4/MD2. This means that hyaluronic acid fragment (4mer) trigger sirtuin induction by binding to TLR4 receptors on the cell surface. Therefore, it became clear that TLR4 is a receptor of the hyaluronic acid fragment, and that the hyaluronic acid fragment, through the action of TLR4, give rise to sirtuin induction in human epidermal keratinocyte stem cells, and hence trigger proliferation of stem cells.

### (Test Example 6) (Sirtuin induction in human epidermal keratinocytes Part 6)

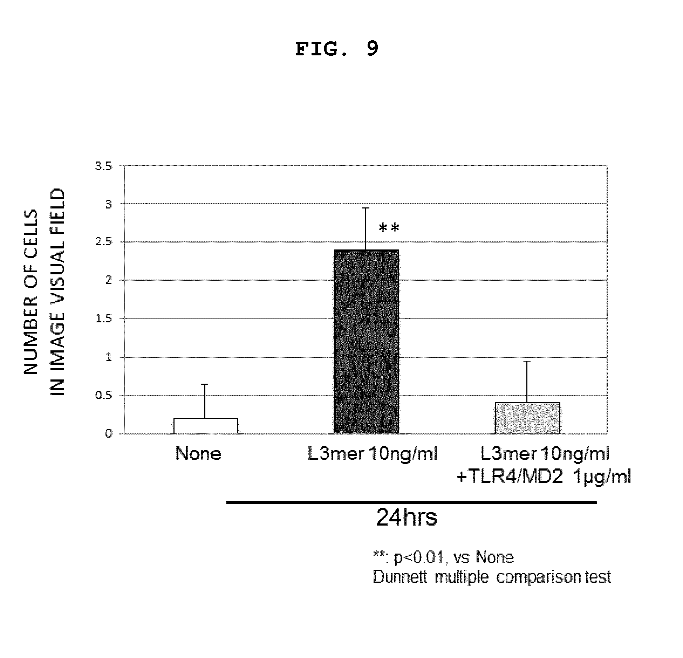

A test similar to Test Example 5 was carried out, using chemically synthesized three-unit size hyaluronic acid fragments (L3mer) in place of hyaluronic acid fragment (4mer). The results are shown in FIG. 9.

As shown in FIG. 9, chemically synthesized three-unit size hyaluronic acid fragments (L3mer) also give rise to sirtuin induction in human epidermal keratinocyte stem cells, and this was substantially completely inhibited through the addition of TLR4/MD2. This means that hyaluronic acid fragment (L3mer) trigger sirtuin induction by binding to TLR4 receptors on the cell surface. Therefore, it was further confirmed that TLR4 in human epidermal keratinocyte stem cells is a receptor of the hyaluronic acid fragment, and that sirtuin induction arises due to action through this receptor, hence triggering proliferation of stem cells.

### (Test Example 7) (Action of L3mer on human neural stem cells)

Human neural stem cells (neurospheres) (trade name Gibco Human Neural Stem Cells (H9-Derived) made by Gibco) were cultured in Neurobasal Media (trade name, made by Gibco) culture medium at 37°C under 5% CO₂, and the cells were recovered by trypsin treatment. The cells were then inoculated into a 96-well plate (100 µL per well) and cultured overnight. Following completion of culture, the culture broth from each well was transferred to Neurobasal Media (trade name, made by Gibco) culture medium to which no hyaluronic acid fragments were added, or to the same medium to which hyaluronic acid fragment (L3mer) had been added to a final concentration of 10 ng/ml, and cultured for 12 days

After 12 days had passed, the cells in each well were double-stained with anti human Nestin antibody and anti human Tubulin antibody. Nestin is a protein that is specifically expressed in neural stem cells, and is a neural stem cell marker. The stained cells were observed under a microscope (made by Nikon), taking photographs of cell morphology and stained images of cells.

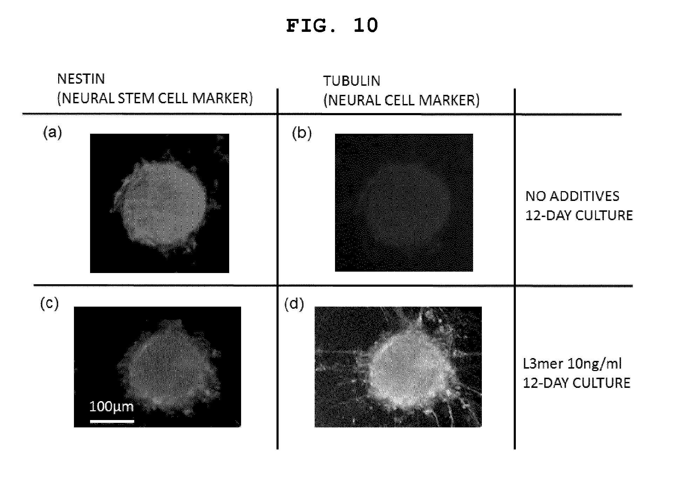

Photographs of stained images are shown in FIG. 10. FIG. 10 (a) shows an anti human Nestin antibody-stained image of human neural stem cells (neurospheres) cultured in culture broth containing no hyaluronic acid fragments. FIG. 10 (b) shows an anti human Tubulin antibody-stained image of human neural stem cells (neurospheres) cultured in culture broth containing no hyaluronic acid fragments. FIG. 10 (c) shows an anti human Nestin antibody-stained image of human neural stem cells (neurospheres) cultured in culture broth containing 10 ng/ml of hyaluronic acid fragment (L3mer). FIG. 10 (d) shows an anti human Tubulin antibody-stained image of human neural stem cells (neurospheres) cultured in culture broth containing 10 ng/ml of hyaluronic acid fragment (L3mer).

As shown in FIG. 10 (a) and (b), in human neural stem cells (neurospheres) cultured in culture broth containing no hyaluronic acid fragments, expression of the human neural stem cell marker Nestin was confirmed; however, the expression of Tubulin, an indicator of cell differentiation, was weak, and in morphological terms, process formation was not confirmed.

On the other hand, as shown in FIG. 10 (c) and (d), in human neural stem cells (neurospheres) cultured in culture broth containing hyaluronic acid fragment (L3mer), expression of both Nestin and Tubulin was confirmed, and in morphological terms, process formation was confirmed. From these results, it may be appreciated that the hyaluronic acid fragment induce neural cell differentiation (process formation and Tubulin expression) while maintaining stemness (Nestin expression) in human neural stem cells (neurospheres).

### (Test Example 8) (Sirtuin induction in human large intestine epithelial cells)

Using a DMEM culture medium (made by Wako Pure Chemical Industries, Ltd.), human large intestine epithelial cells (HT29 cells) were cultured by usual methods at 37°C under 5% CO₂. The culture broth was transferred to DMEM culture medium containing no hyaluronic acid fragments; to DMEM culture medium containing hyaluronic acid fragment (4mer) in a final concentration of 100 ng/ml; or to DMEM culture medium containing the hyaluronic acid fragment (4mer) plus TLR4/MD2 in a final concentration of 10 µg/ml, and cultured for 24 hours.

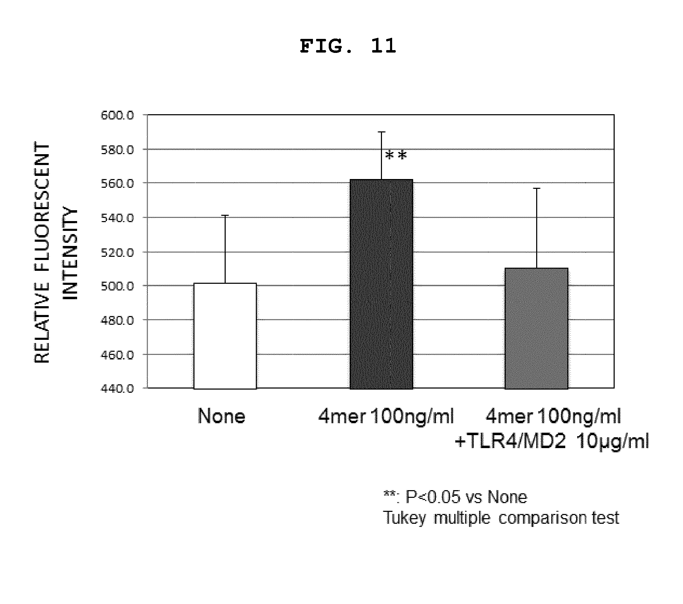

After 24 hours had passed, the cells of each well were stained with human anti SIRT1 antibody, and the immunostaining intensity was measured with an Array Scan scanning device (made by Thermo Scientific). Results are shown in FIG. 11.

As shown in FIG. 11, treatment with the hyaluronic acid fragment gave rise to sirtuin induction in human large intestine epithelial cells (HT29 cells) as well, and this was substantially completely inhibited through the addition of TLR4/MD2. Therefore, it is clear that, in human large intestine epithelial cells (HT29 cells) as well, TLR4 is a receptor for the hyaluronic acid fragment, and that sirtuin induction is triggered by action through this receptor.

### (Test Example 9) (Action on NF-kappaB expression in human large intestine epithelial cells Part 1)

It was investigated whether the expression of NF-kappaB in human large intestine epithelial cells (HT29 cells) when subjected to TNF-α treatment is affected by treatment with the hyaluronic acid fragment. NF-kappaB is a transcription factor involved in inflammation, tissue destruction, and the like.

DMEM culture medium (made by Wako Pure Chemical Industries, Ltd.) to which TNF-α and the hyaluronic acid fragment were added, and DMEM culture medium from which these were omitted, were prepared, and human large intestine epithelial cells were treated for 24 hours in these culture media. Thereafter, the cells were stained with an antibody that recognizes activated (phosphorylated) NF-kappaB.

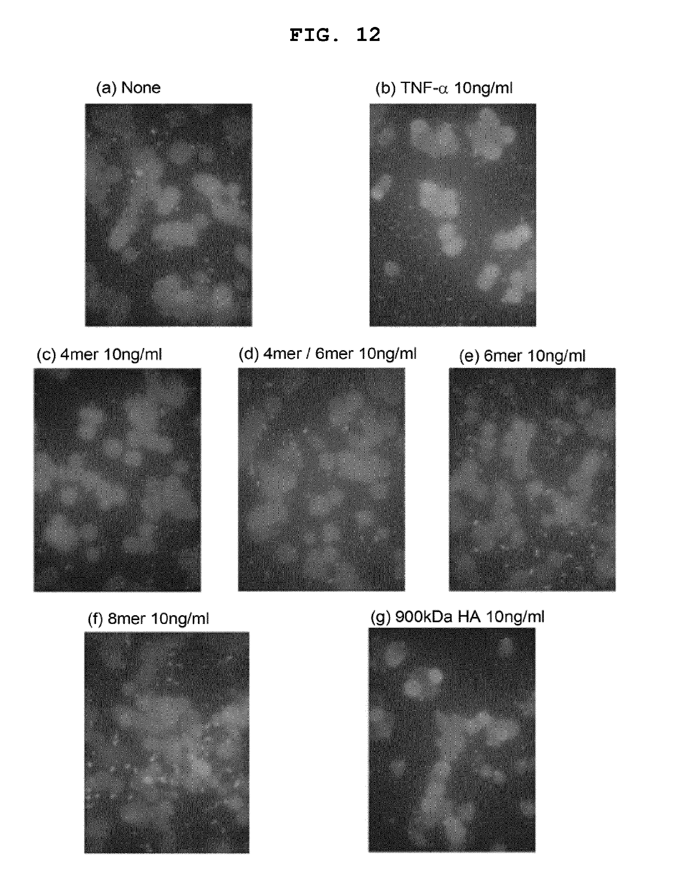

FIG. 12 shows photographs of human large intestine epithelial cells fluorescently-stained with NF-kappaB. FIG. 12 (a) shows human large intestine epithelial cells cultured in a culture broth to which neither hyaluronic acid fragments nor TNF-α were added. FIG. 12 (b) shows human large intestine epithelial cells cultured in a culture broth treated with TNF-α only, with no hyaluronic acid fragments added. FIG. 12 (c) shows human large intestine epithelial cells cultured in a culture broth containing TNF-α and 10 ng/ml of four-unit hyaluronic acid fragment (4mer). FIG. 12 (d) shows human large intestine epithelial cells cultured in a culture broth containing TNF-α and 10 ng/ml of four-unit and six-unit hyaluronic acid fragments (5 ng/ml each of the 4mer and 6mer). FIG. 12 (e) shows human large intestine epithelial cells cultured in a culture broth containing TNF-α and 10 ng/ml of six-unit hyaluronic acid fragment (6mer). FIG. 12 (f) shows human large intestine epithelial cells cultured in a culture broth containing TNF-α and 10 ng/ml of eight-unit hyaluronic acid fragment (8mer). FIG. 12 (g) shows human large intestine epithelial cells cultured in a culture broth treated with TNF-α and containing 10 ng/ml of 900 kDa hyaluronic acid.

From a comparison of FIG. 12 (a) and FIG. 12 (b), it may be appreciated that the expression level of NF-kappaB increases with TNF-α treatment of human large intestine epithelial cells. Because NF-kappaB is a protein having increased expression levels with aging, it is an indicator protein for aging (Salminen A, Kaarniranta K. "Genetics vs. entropy: longevity factors suppress the NF-kappaB-driven entropic aging process" Ageing Res. Rev. 2010 Jul; 9(3): 298-314). That is, in FIG. 12 (b), aging of cells is advanced by TNF-α treatment. On the other hand, from FIG. 12 (c) to FIG. 12 (f) it may be appreciated that, in spite of TNF-α treatment, expression of NF-kappaB is suppressed through addition of hyaluronic acid fragments. In FIG. 12 (g), 900 kDa high-molecular weight hyaluronic acid was added. In FIG. 12 (g) as well, the expression level of NF-kappaB was observed to be reduced, but the extent of the reduction was not as much as in cases in which hyaluronic acid fragments were added. From these results, it may be appreciated that, in human large intestine epithelial cells cultured in culture broth containing hyaluronic acid fragments, the expression level of NF-kappaB, which is triggered by TNF-α treatment, can be inhibited.

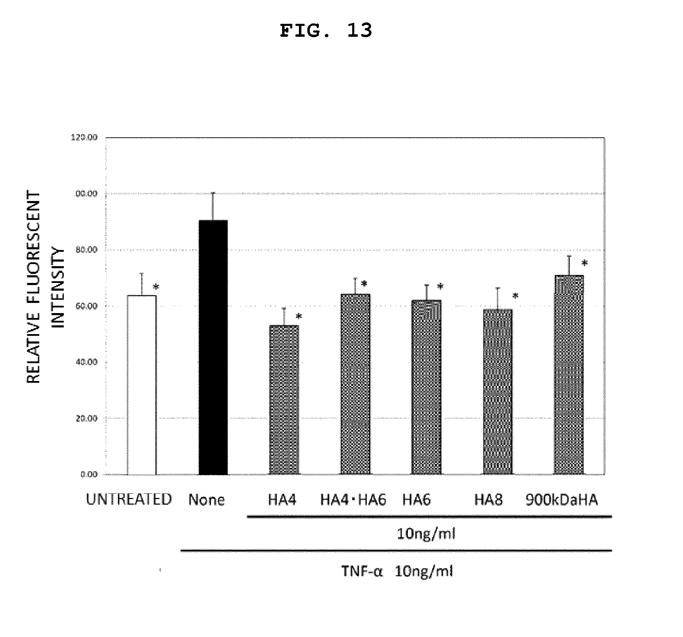

FIG. 13 shows a graph of fluorescent intensity of fluorescently-stained NF-kappaB. The vertical axis of the graph indicates fluorescent intensity. It may be appreciated that, in cells to which four- to eight-unit hyaluronic acid fragments were added, the expression level of NF-kappaB is reduced in statistically significant fashion. It may be further appreciated that the increase in expression level of NF-kappaB is inhibited to the greatest extent by four-unit hyaluronic acid fragment.

From the above results, it was concluded that aging of cells can be inhibited in cases in which cells are cultured in cell culture broth to which the hyaluronic acid fragment has been added.

### (Test Example 10) (Action on NF-kappaB expression in human large intestine epithelial cells Part 2)

Using a DMEM culture medium (made by Wako Pure Chemical Industries, Ltd.), human large intestine epithelial cells (HT29 cells) were cultured by usual methods at 37°C under 5% CO₂. The culture broth was transferred to DMEM culture medium containing no added hyaluronic acid fragments; to DMEM culture medium containing TNF-α added to a final concentration of 100 ng/ml; to DMEM culture medium containing the TNF-α plushyaluronic acid fragment (4mer) added to a final concentration of 100 ng/ml; or to DMEM culture medium containing the TNF-α and hyaluronic acid fragment (4mer), plus TLR4/MD2 added to a final concentration of 10 µg/ml, and cultured for 24 hours.

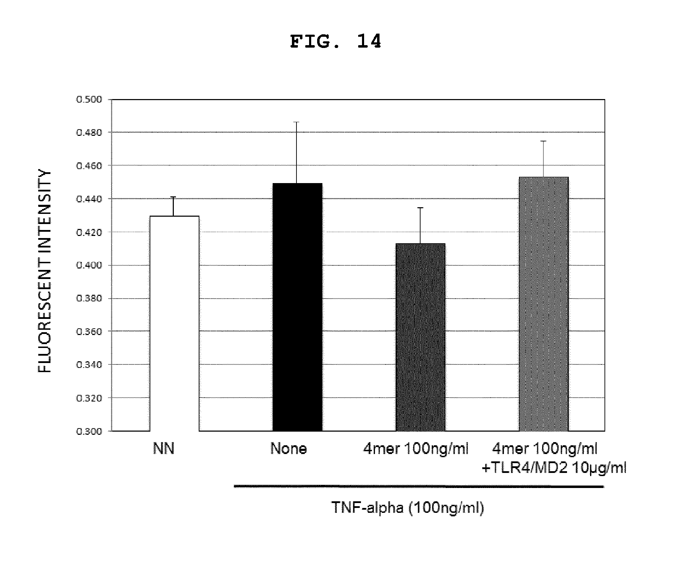

After 24 hours had passed, the cells of each well were stained with the anti phosphorylated NF-kappaB antibody, and the immunostaining intensity was measured with an Array Scan scanning device (made by Thermo Scientific). Results are shown in FIG. 14.

As shown in FIG. 14, in this test example as well, expression of NF-kappaB in human large intestine epithelial cells (HT29 cells) induced through TNF-α treatment was suppressed through treatment with the hyaluronic acid fragment. This was substantially completely inhibited through the addition of TLR4/MD2. Therefore, it is clear that TLR4 in human large intestine epithelial cells (HT29 cells) is a receptor for the hyaluronic acid fragment, and that suppression of NF-kappaB expression is triggered by action via this receptor.

### (Test Example 11) (Effect of action on corneal damage Part 1)

The effect of action of the hyaluronic acid fragment on corneal damage was investigated. Test ophthalmic solutions were prepared as follows.

### (Test ophthalmic solutions)

- Physiological saline in which hyaluronic acid fragments (a mixture of the L3mer and 4mer in equal concentration) were dissolved to a final concentration of 0.1 w/v%
- An ophthalmic solution containing 0.1 w/v% of 500,000-1,200,000 average-molecular weight hyaluronic acid as an active ingredient
- Physiological saline

For the test, six rabbits aged 22-24 months (with body weight of 3-4 kg) were used. Filter paper (disks 1 cm in diameter) moistened with 0.05 ml of n-heptanol were placed in both eyes of the six rabbits, inflicting wounds to the cornea of the rabbits' eyes. Thereafter, the test ophthalmic solutions were dripped in 0.05 ml into one eye of each rabbit after 2 hours, 6 hours, 24 hours, 27 hours and 30 hours, respectively. At the same time, physiological saline was dripped in 0.05 ml into the other eye of each rabbit, as a control.

24 hours and 48 hours after inflicting a wound to the cornea, the corneas of the rabbits' eyes were photographed with a Nikon camera. Utilizing the fact that fluorescent agent FITC adheres to wound regions, this was dripped into the rabbits' eyes, and the surface area of the wound region was measured by image analysis software ("NIH Image").

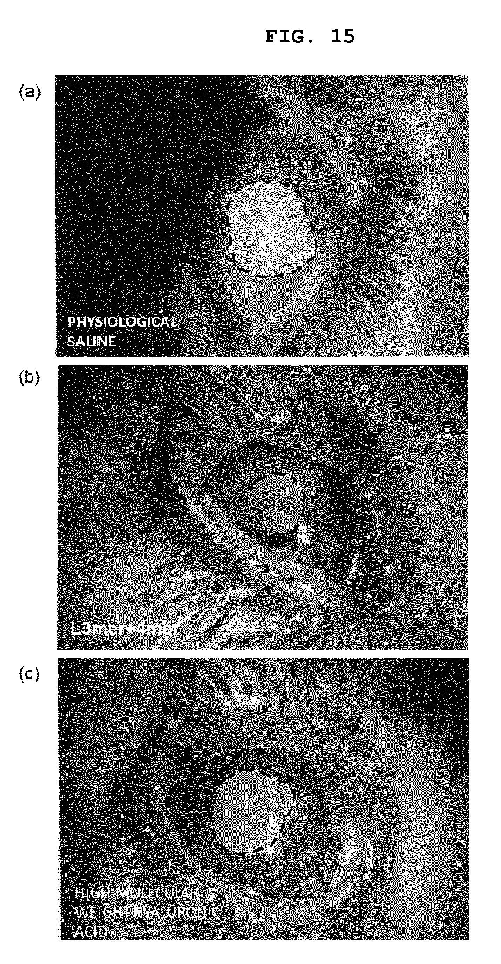

FIG. 15 shows photos taken of the condition of the cornea in the eye of a rabbit, 24 hours after inflicting injury to the cornea. The wound region in the rabbit's eye is shown by broken lines. FIG. 15 (a) shows the condition of the cornea of the rabbit's eye after 24 hours, in the case of dripping in physiological saline. The wound region was larger than the pupil part of the rabbit's eye, extending over the entirety thereof. FIG. 15 (b) shows the condition of the cornea of the rabbit's eye after 24 hours, in the case of dripping in the ophthalmic solution containing the hyaluronic acid fragments. The wound region was confined within an area smaller than the pupil part of the rabbit's eye. Therefore, after 24 hours, the wound region of the cornea of the rabbit's eye was smaller in the case of having dripped in the ophthalmic solution containing the hyaluronic acid fragments, as compared with the case of having dripped in physiological saline. FIG. 15 (c) shows the condition of the cornea of the rabbit's eye after 24 hours, in the case of having dripped in the ophthalmic solution containing 500,000-1,200,000 average-molecular weight hyaluronic acid. While the wound region is slightly smaller as compared with the case of having dripped in physiological saline, the shrinkage is not very great.

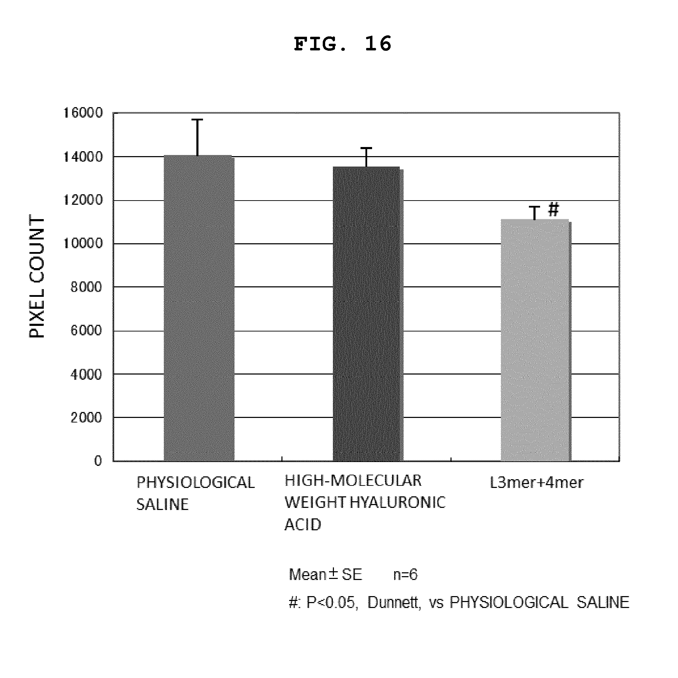

FIG. 16 shows a graph of surface area of the wound region 24 hours after inflicting injury to the cornea. In the case of having dripped in physiological saline, the average surface area of the wound region was 14,000 pixels, whereas in the case of having dripped in the ophthalmic solution containing the hyaluronic acid fragments, the average surface area of the wound region was a smaller value of 11,000 pixels. In the case of having dripped in the ophthalmic solution containing 500,000-1,200,000 average-molecular weight hyaluronic acid, the average surface area was 13,500 pixels, and the reduction in the value was not statistically significant.

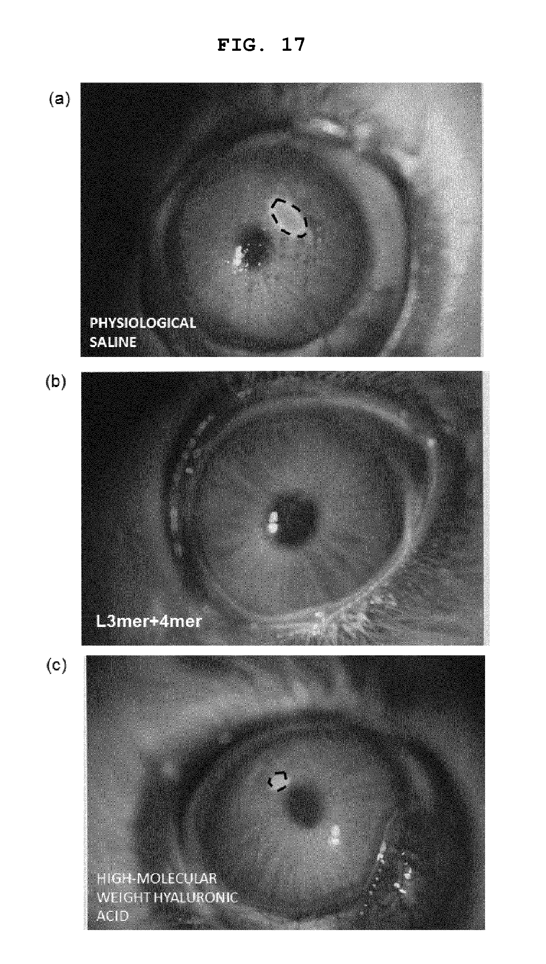

FIG. 17 shows photos taken of the condition of the cornea in the eye of a rabbit, 48 hours after inflicting injury to the cornea. The wound region in the rabbit's eye is shown by broken lines. FIG. 17 (a) shows the condition of the cornea of the rabbit's eye after 48 hours, in the case of dripping in physiological saline. The surface area of the wound region was smaller in comparison with the 24-hour point, but the wound region remained. FIG. 17 (b) shows the condition of the cornea of the rabbit's eye after 48 hours, in the case of dripping in the ophthalmic solution containing the hyaluronic acid fragments. In the case of dripping in the ophthalmic solution containing the hyaluronic acid fragments, the wound region had disappeared completely after 48 hours. Therefore, it may be appreciated that whereas in the case of dripping in physiological saline, the wound region still remained even after 48 hours, in the case of dripping in the ophthalmic solution containing the hyaluronic acid fragments, the wound region had disappeared completely after 48 hours. FIG. 17 (c) shows the condition of the cornea of the rabbit's eye after 48 hours, in the case of dripping in the ophthalmic solution containing 500,000-1,200,000 average-molecular weight hyaluronic acid. While the surface area is smaller in comparison with the case of dripping in physiological saline, the wound region remained.

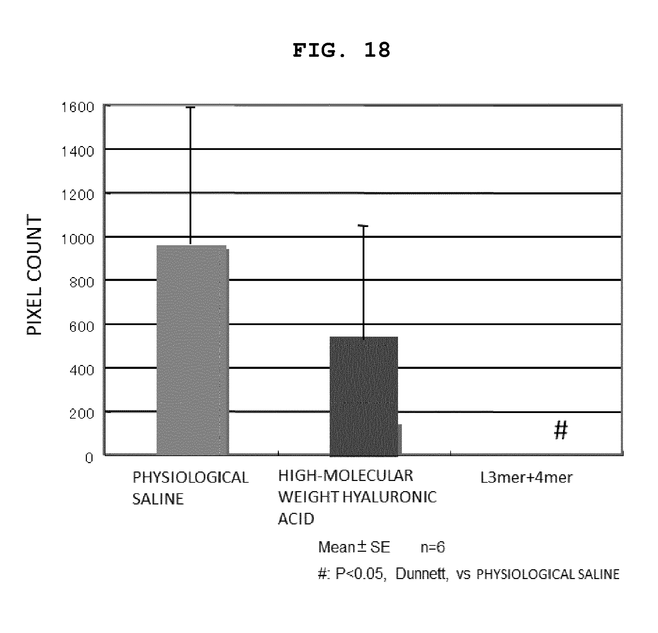

FIG. 18 shows a graph of surface area of the wound region 48 hours after inflicting injury to the cornea. In the case of having dripped in physiological saline, the average surface area of the wound region was 950 pixels, whereas in the case of having dripped in the ophthalmic solution containing the hyaluronic acid fragments, the average surface area of the wound region was 0 pixels, and the wound region had completely disappeared. In the case of having dripped in the ophthalmic solution containing 500,000-1,200,000 average-molecular weight hyaluronic acid, the average surface area was 520 pixels, and the reduction in the value was not statistically significant.

From the above results, it was found that, the case of dripping in the ophthalmic solution containing the hyaluronic acid fragment, the recovery time of the cornea of the injured eye was shorter. That is, it was found that the hyaluronic acid fragment repair tissue.

### (Test Example 12) (Effect of action on corneal damage Part 2)

In the same manner as in Test Example 11, injuries were inflicted to corneas of rabbits, test solutions were dripped in subsequent to injury, and 33 hours after inflicting injury, corneal tissue was harvested from the eyes of the rabbits. The corneal tissue was fixed with para-formaldehyde, followed by preparation of paraffin-embedded specimens and frozen embedded specimens. Thin slices were prepared from the paraffin-embedded specimens, and the thin slices were subjected to hematoxylin-eosin staining. The stained corneal tissue was examined under an Olympus microscope, recording the condition of the corneal epithelium of the corneal tissue.

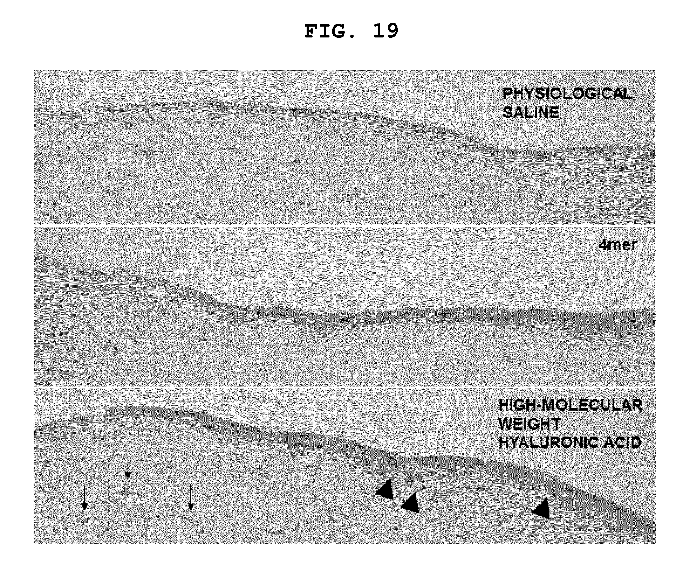

FIG. 19 shows hematoxylin-eosin stains of corneal tissue of the eyes of rabbits after 33 hours. FIG. 19 (a) shows a hematoxylin-eosin stain of corneal tissue of the eye of a rabbit after 33 hours, in the case of dripping in physiological saline. The dark-colored section shows the corneal epithelium; the corneal epithelium was found to be extremely thin. FIG. 19 (b) shows a hematoxylin-eosin stain of corneal tissue of the eye of a rabbit after 33 hours, in the case of dripping in the ophthalmic solution containing the hyaluronic acid fragment. In this case, it was found that the corneal epithelium had formed more thickly across a wide area. Moreover, it was found that a plurality of cellular layers had formed on the corneal epithelium.

FIG. 19 (c) shows a hematoxylin-eosin stain of corneal tissue of the eye of a rabbit after 33 hours, in the case of dripping in the ophthalmic solution containing 500,000-1,200,000 average-molecular weight hyaluronic acid. In this case, vacuoles in the corneal epithelium (shown by arrows in the drawing), and activation of keratocytes (which are normally flattened, but appeared fusiform) (shown by other arrows in the drawing) were verified. This result indicates that high-molecular weight hyaluronic acid produces some sort of damage to tissue. With the hyaluronic acid fragment, such tissue damage was not observed, making it clear that the hyaluronic acid fragment is superior as a tissue repairing agent for treating corneal injury.

From the above results, it was found that the hyaluronic acid fragment promotes cell proliferation in wound regions and peripheral regions thereof. Moreover, it was found that in the case of dripping in the ophthalmic solution containing the hyaluronic acid fragment, formation of the corneal epithelium advanced, and a plurality of cellular layers formed. It was thereby found that, in the case of dripping in the ophthalmic solution containing the hyaluronic acid fragment, cell differentiation is induced in wound regions. That is, it was found that in addition to promoting cell proliferation, the hyaluronic acid fragment can also induce cell differentiation and repair tissue.

### (Test Example 13) (Induction of hepatocyte growth factor in human epidermal keratinocytes)

Human epidermal keratinocytes (normal human keratinocytes from an Asian female (age 27)) were cultured respectively in a culture medium containing 10 ng/ml of four-unit hyaluronic acid fragment (4mer), and a culture medium containing no hyaluronic acid fragments (untreated), for 24 hours. For the culture medium, a serum-free medium for use with normal human keratinocytes, made by DS Pharma Biomedical Co. Ltd. was employed. After culture, the cells were recovered and analyzed with a DNA microarray (DNA chip).

Table 3 shows the intensity of fluorescence (Cy5) of normal human keratinocytes cultured in cell culture broth to which the hyaluronic acid fragment were added, and those cultured in cell culture broth to which no hyaluronic acid fragments were added.

**[Table 3]**

| Hepatocyte growth factor expression in keratinocytes (mRNA) | | |
|---|---|---|
| | Untreated | 4mer 10ng/ml |
| Fluorescent intensity | 95.89 | 200.40 |

From the results in Table 3, it was found that in the case of cells cultured in cell culture broth to which the hyaluronic acid fragment was added, the expression level of hepatocyte growth factor increased two-fold.

### (Test Example 14) (Effect of action on liver of aged rat Part 1)

The effect of action of the hyaluronic acid fragment on the liver of aged rat was investigated. The following test solutions were prepared.

### (Test solutions)

- Organ preservation solution (University of Wisconsin solution: the standard solution used for organ preservation)
- The above organ preservation solution, into which hyaluronic acid fragment (4mer) was dissolved to a final concentration of 100 ng/ml
- The above organ preservation solution, into which hyaluronic acid fragment (L3mer) was dissolved to a final concentration of 100 ng/ml
- The above organ preservation solution, into which hyaluronic acid fragment (L3mer) was dissolved to a final concentration of 100 ng/ml, then dissolving TLR4/MD2 into the above organ preservation solution, to a final concentration of 10 µg/ml.

SD rats aged 24 months (aged rats) were injected with the test solutions from the hepatic portal, and after perfusion of 7.5 ml, were preserved for six hours in the same solution at 37°C. Slices were prepared from blocks prepared by freezing of the livers embedded in OCT compound, and were subjected to microscope examination.

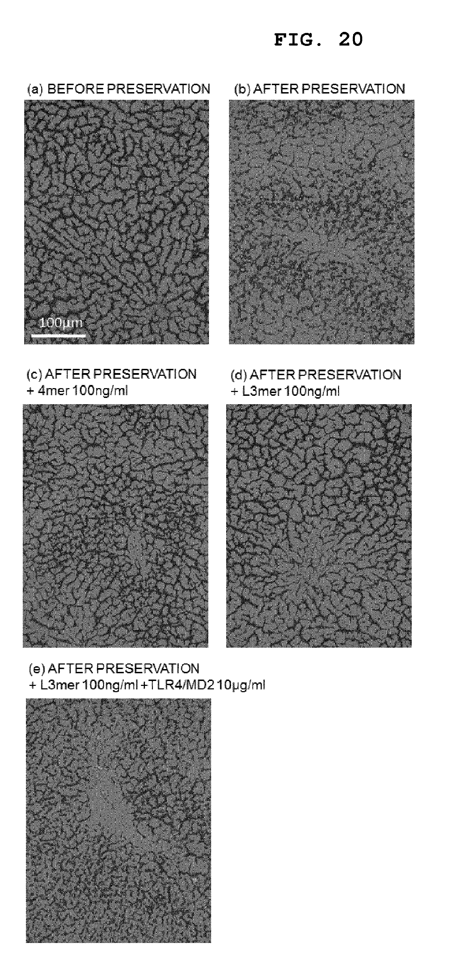

Microscope image photographs are shown in FIG. 20. FIG. 20 (a) shows the condition of an untreated liver tissue slice prior to preservation. Prior to preservation, distinct sinusoids could be observed. FIG. 20 (b) shows the condition of a liver tissue slice after preservation in organ preservation solution with no additives. When preserved in the organ preservation solution, the sinusoids became indistinct (sinusoidal constriction), and numerous blebs (bubbles) appeared (cellular damage). Blebs (bubbles) that appear in ischemic liver tissue arise as a result of an activation of Ca ion-dependent proteolytic enzymes and a disorder of the cytoskeletal system, in which lowered ATP levels due to ischemia lead to effusion of Ca ions from the mitochondria to the cellular cytoplasm. In the course of passing through these changes, the liver cells experience cell death. Such changes have been confirmed to occur in clinical conditions such as pharmaceutical toxicity, aging, and the like, and in concanavalin A-induced liver damage.

FIG. 20 (c) shows the condition of a liver tissue slice after preservation in organ preservation solution to which hyaluronic acid fragment (4mer) was added. When preserved in organ preservation solution containing hyaluronic acid fragment (4mer), sinusoidal constriction and appearance of blebs were suppressed. FIG. 20 (d) shows the condition of a liver tissue slice after preservation in organ preservation solution to which hyaluronic acid fragment (L3mer) was added. When preserved in organ preservation solution containing hyaluronic acid fragment (L3mer), sinusoidal constriction and appearance of blebs were suppressed. FIG. 20 (e) shows the condition of a liver tissue slice after preservation in organ preservation solution to which hyaluronic acid fragment (L3mer) and TLR4/MD2 were added. In this case, the sinusoids became indistinct (sinusoidal constriction), and numerous blebs (bubbles) appeared.

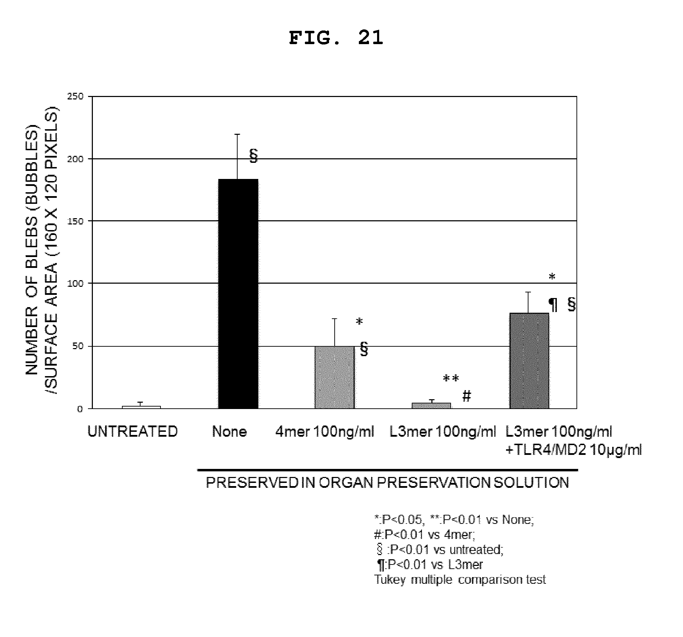

FIG. 21 is a graph summarizing bleb (bubble) count per unit surface area. As shown in FIG. 21, the effect of suppressing the appearance of blebs was higher with L3mer treatment than with 4mer treatment. This is attributed to the greater ability of the L3mer, due to its lower molecular weight, to infiltrate tissue as compared with the 4mer, so as to more effectively suppress the appearance of blebs. The effect of the L3mer in suppressing the appearance of blebs was reduced by the addition of TLR4/MD2. It was clearly indicated thereby that the effect of suppressing damage to the liver during preservation is due to the action of the hyaluronic acid fragment via TLR4.

### (Test Example 15) (Effect of action on liver in aged rat Part 2)

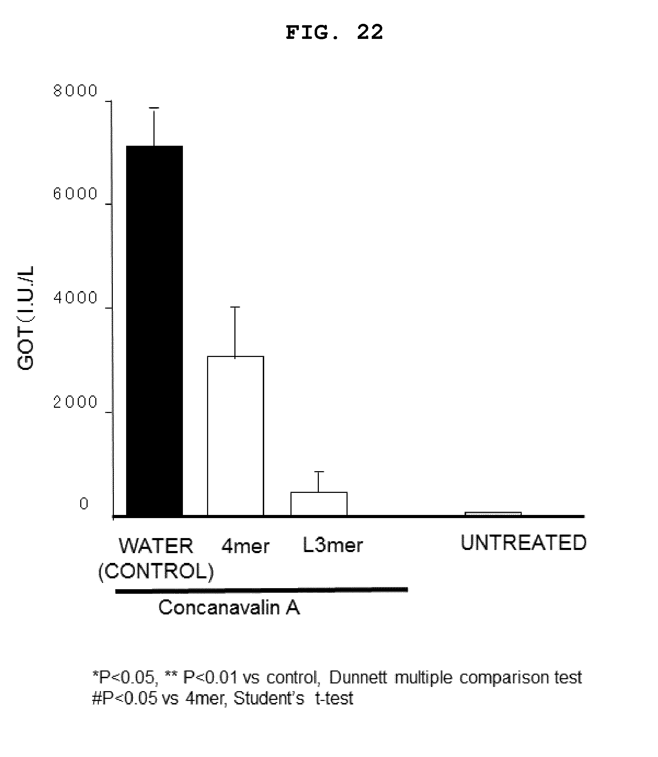

The effect of action of the hyaluronic acid fragment on the liver of aged rat was investigated. Specifically, 20-month old (aged) female Balb/c mice were administered through the caudal vein with concanavalin A (20 mg/kg of body weight) one time, as a liver damage model. Subsequent to administration of concanavalin A, hyaluronic acid fragment (4mer or L3mer) were administered orally, and the effect thereof was investigated. Impairment of liver function was evaluated after 24 hours, by drawing blood and measuring the GOT and GPT values in the blood. The results for GOT values in the blood are shown in FIG. 22.

As shown in FIG. 22, impairment of liver function due to administration of concanavalin A was ameliorated through administration of the hyaluronic acid fragment. The effect of doing so was markedly better with the L3mer than with the 4mer. This is attributed to the greater ability of the L3mer, due to its lower molecular weight, to infiltrate tissue as compared with the 4mer, so as to more effectively suppress impairment of liver function due to administration of concanavalin A. The results for GPT values showed a similar trend.

### (Test Example 16) (Action on human large intestine epithelial cells subjected to induced aging Part 1)

Human large intestine epithelial cells (HT29 cells) were experienced cellular aging (decline in mitochondrial activity due to injury, or a drop in the MTS value once cell death occurs) due to stimulation by the hydrogen peroxide when treated with hydrogen peroxide, and it was investigated whether this process is affected by treatment with the hyaluronic acid fragment. Specifically, the following test was performed.

Using a DMEM culture medium (made by Wako Pure Chemical Industries, Ltd.), human large intestine epithelial cells (HT29 cells) were cultured by usual methods at 37°C under 5% CO₂. Hydrogen peroxide was added to the culture medium to a final concentration of 10 µM to bring about stimulation for a 15-minute period, and the culture broth was then transferred to DMEM culture medium containing no hyaluronic acid fragments; to DMEM culture medium containing hyaluronic acid fragment (4mer) in a final concentration of 100 ng/ml; or to DMEM culture medium containing the hyaluronic acid fragment (4mer) plus TLR4/MD2 in a final concentration of 10 µg/ml, and cultured for 3 hours.

After 3 hours had passed, an MTS assay was performed. On the basis of the action, in which MTS is converted to formazan by dehydrogenation in the mitochondria of the cell, OD 490 nm was measured to measure mitochondrial function.

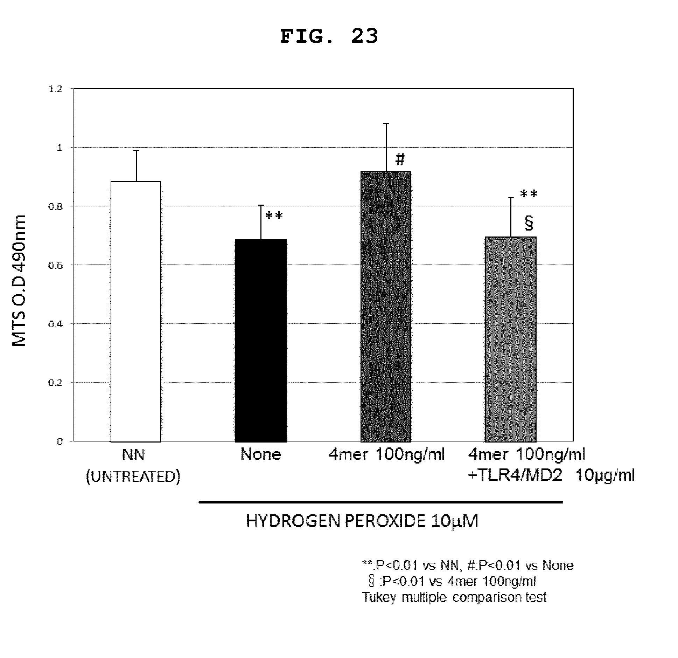

As shown in FIG. 23, hyaluronic acid fragment (4mer) suppressed cellular aging (decline in mitochondrial activity due to injury, or a drop in the MTS value once cell death occurs) due to stimulation by the hydrogen peroxide. Because this action was inhibited by addition of TLR4/MD2 to the culture medium, it was further determined that this action is due to binding of the hyaluronic acid fragment to TLR4 on cell surfaces. It was therefore clear that TLR4 in human large intestine epithelial cells (HT29 cells) is a receptor for the hyaluronic acid fragment, and that the effect of suppressing cellular aging is produced through action via this receptor.

### (Test Example 17) (Action on human large intestine epithelial cells subjected to induced aging Part 2)

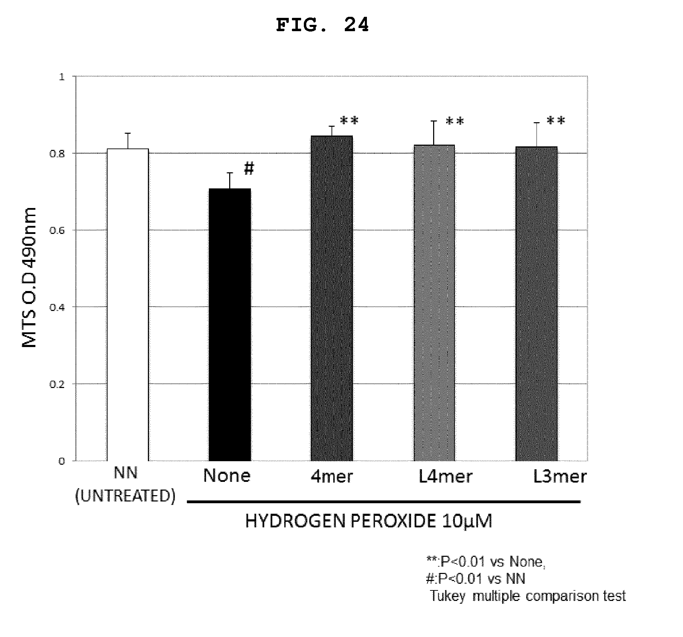

Using hyaluronic acid fragment (4mer), hyaluronic acid fragment (L4mer) and hyaluronic acid fragment (L3mer) as test substances, tests similar to those of Test Example 16 were performed, except that the culture time following transfer to the culture medium containing the test substance was one hour. Results are shown in FIG. 24.

As shown in FIG. 24, the chemically synthesized L4mer and L3mer, like the 4mer, showed effect in suppressing cellular aging.

### (Test Example 18) (Action on human large intestine epithelial cells subjected to induced aging Part 3)

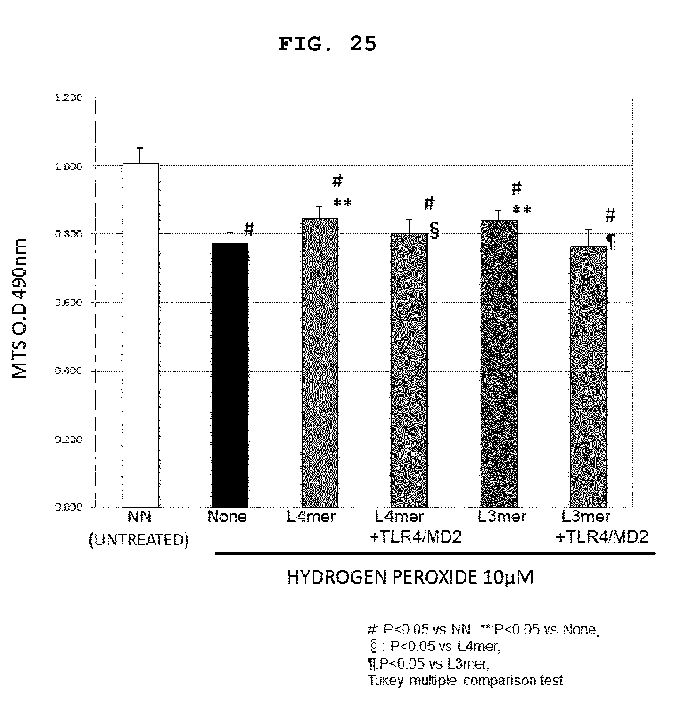

Tests similar to those of Test Example 16 were performed, except for using hyaluronic acid fragment (L4mer), and hyaluronic acid fragment (L3mer) as test substances. Results are shown in FIG. 25.

As shown in FIG. 25, the chemically synthesized L4mer and L3mer, like the 4mer, showed effect in suppressing cellular aging. Because this action was inhibited by the addition of TLR4/MD2 to the culture medium, it was determined that the aging suppressant effect of the L4mer and L3mer is produced via TLR4.

### (Test Example 19) (Action on human oral epithelial cells subjected to induced aging)

It was investigated whether the process , in whichmitochondrial function of human oral epithelial cells (Ca9-22 cells) declines when treated with hydrogen peroxide, is affected by treatment with the hyaluronic acid fragment. Specifically, the following test was performed.

Using a DMEM culture medium (made by Wako Pure Chemical Industries, Ltd.), human oral epithelial cells (Ca9-22 cells) were cultured by usual methods at 37°C under 5% CO₂. Hydrogen peroxide was added to the culture medium to a final concentration of 1 µM to bring about stimulation for a 15-minute period, and the culture broth was then transferred to DMEM culture medium containing no hyaluronic acid fragments; to DMEM culture medium containing hyaluronic acid fragment (L3mer) in a final concentration of 100 ng/ml; to DMEM culture medium containing hyaluronic acid fragment (4mer) in a final concentration of 100 ng/ml; or to DMEM culture medium containing 900 kDa hyaluronic acid in a final concentration of 100 ng/ml, and cultured for 3 hours.

After 3 hours had passed, an MTS assay was performed. On the basis of the action, in which MTS is converted to formazan by dehydrogenation in the mitochondria of the cell, OD 490 nm was measured to measure mitochondrial function.

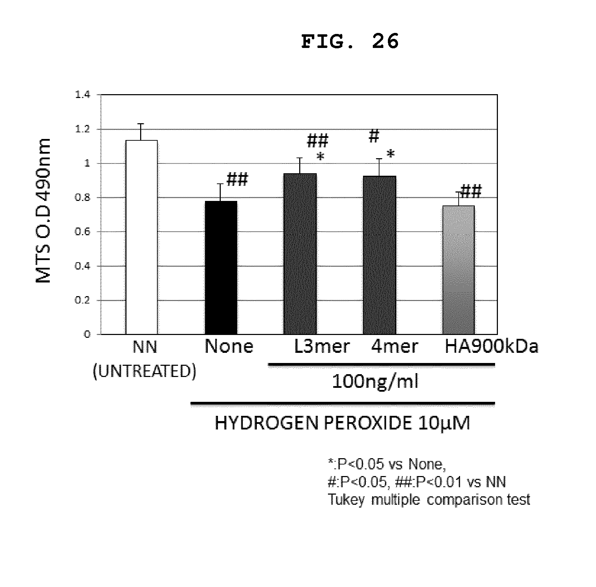

As shown in FIG. 26, hyaluronic acid fragment (L3mer or 4mer) was clearly found to suppress cellular aging in human oral epithelial cells due to stimulation by hydrogen peroxide. On the other hand, no action of suppressing cellular aging was obtained with 900 kDa hyaluronic acid.

### (Test Example 20) (Action on human neural stem cells subjected to induced aging)

It was investigated whether the process, in which human neural stem cells (neurospheres) experienced cellular aging (vacuolar degeneration and hypertrophy) when treated with hydrogen peroxide, is affected by treatment with the hyaluronic acid fragment. Specifically, the following test was performed.

Human neural stem cells (neurospheres) (trade name Gibco Human Neural Stem Cells (H9-Derived) made by Gibco) were cultured in Neurobasal Media (trade name, made by Gibco) culture medium at 37°C under 5% CO₂. Hydrogen peroxide was added to the culture medium to a final concentration of 10 µM to bring about stimulation for a 15-minute period, and the culture broth was then transferred to Neurobasal Media culture medium (made by Gibco) containing no hyaluronic acid fragments; to the same culture medium containing hyaluronic acid fragment (L3mer) in a final concentration of 100 ng/ml; or to the same culture medium containing hyaluronic acid fragment (L3mer) plus TLR4/MD2 added to a final concentration of 10 µg/ml, and cultured for 24 hours.

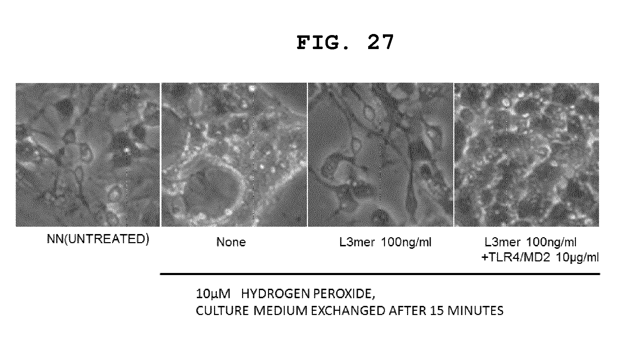

After 24 hours, the cells were examined with an inverted microscope. Photographs of the microscope images are shown in FIG. 27.

As shown in FIG. 27, hydrogen peroxide-induced cellular aging (vacuolar degeneration and hypertrophy) was suppressed by hyaluronic acid fragment (L3mer). Because this action was inhibited by addition of TLR4/MD2 to the culture medium, it was further determined that this action is due to binding of the hyaluronic acid fragment to TLR4 on cell surfaces. It was therefore clear that TLR4 in human neural stem cells (neurospheres) is a receptor for the hyaluronic acid fragment, and that the effect of suppressing cellular aging is produced through action via this receptor.

### (Test Example 21) (Action on rat bone marrow mesenchymal stem cells subjected to induced aging)

It was investigated whether the process, in which rat bone marrow mesenchymal stem cells experienced cellular aging (increased cell death) when treated with hydrogen peroxide, is affected by treatment with the hyaluronic acid fragment. Specifically, the following test was performed.

Using a DMEM culture medium (made by Wako Pure Chemical Industries, Ltd.), rat bone marrow mesenchymal stem cells were cultured by usual methods at 37°C under 5% CO₂. Hydrogen peroxide was added to the culture medium to a final concentration of 1 µM to bring about stimulation for a 15-minute period, and the culture broth was then transferred to DMEM culture medium containing no added hyaluronic acid fragments; to DMEM culture medium containing hyaluronic acid fragment (L3mer) added to a final concentration of 100 ng/ml; or to DMEM culture medium containing the hyaluronic acid fragment (L3mer) plus TLR4/MD2 added to a final concentration of 10 µg/ml, and cultured for 2 hours.

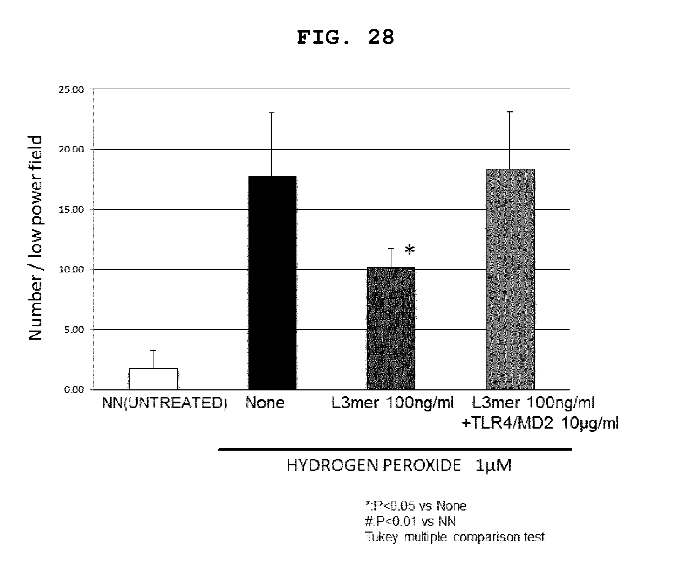

After 2 hours had passed, PI (propidium oxide), which emits red fluorescence upon uptake into dead cells, was added to the culture medium to a final concentration of 5 µg/ml, and after incubation for 15 minutes, the condition of appearance of dead cells was examined with a fluorescence microscope. Average numbers of dead cells within the unit visual field are shown in FIG. 28.

As shown in FIG. 28, hydrogen peroxide-induced cellular aging (increased cell death) was suppressed by hyaluronic acid fragment (L3mer). Because this action was inhibited by addition of TLR4/MD2 to the culture medium, it was further determined that this action is due to binding of the hyaluronic acid fragment to TLR4 on cell surfaces. It was therefore clear that TLR4 in rat bone marrow mesenchymal stem cells is a receptor for the hyaluronic acid fragment, and that the effect of suppressing cellular aging is produced through action via this receptor.

### (Test Example 22) (Detection of keratinocyte marker in human epidermal keratinocytes Part 1)

Human epidermal keratinocytes were stimulated in the same manner as in Test Example 1, except for using the 4mer or the L3mer as the hyaluronic acid fragment, and further adding TLR4/MD2 to a final concentration of 1 µg/ml.

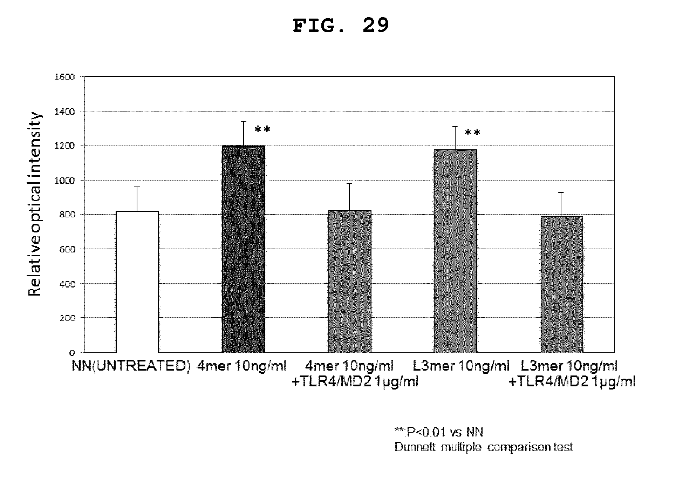

The cells in each well were stained with anti keratin K14 antibody, and the immunostaining intensity was measured with an Array Scan scanning device (made by Thermo Scientific). Keratin K14 is a marker of basal layer keratinocytes. The results are shown in FIG. 29.

As shown in FIG. 29, cells with high expression of Keratin K14 increased through treatment with hyaluronic acid fragment (the 4mer or L3mer). This indicates the cell differentiation was induced by the 4mer or the L3mer. Because this action was also inhibited by addition of TLR4/MD2 to the culture medium, it was further determined that the keratinocyte inducing effect of the 4mer and the L3mer is produced via TLR4.

### (Test Example 23) (Detection of keratinocyte marker in human epidermal keratinocytes Part 2)

Human epidermal keratinocytes were stimulated in the same manner as in Test Example 1, except for using the 4mer or the L3mer as the hyaluronic acid fragment, and further adding TLR4/MD2 to a final concentration of 1 µg/ml.

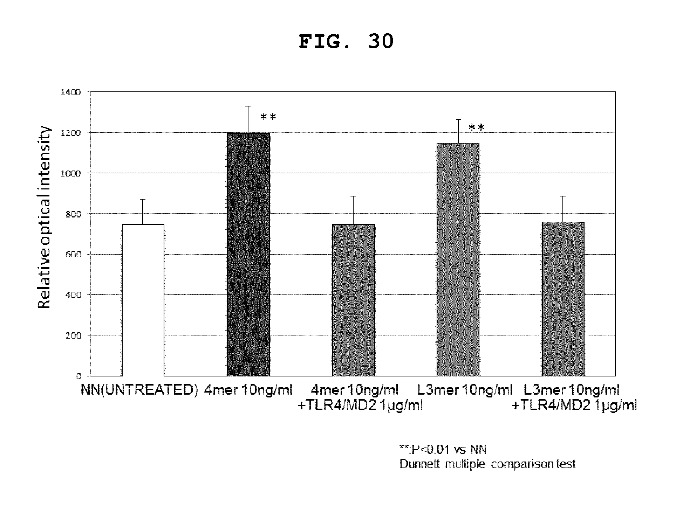

The cells in each well were stained with the anti filaggrin antibody, and the immunostaining intensity was measured with an Array Scan scanning device (made by Thermo Scientific). Filaggrin, which is produced by granular layer keratinocytes, is a marker of mature keratinocytes. The results are shown in FIG. 30.

As shown in FIG. 30, cells with high expression of filaggrin increased through treatment with hyaluronic acid fragment (the 4mer or L3mer). This indicates the cell differentiation was induced by the 4mer or the L3mer. Because this action was also inhibited by addition of TLR4/MD2 to the culture medium, it was further demonstrated that the keratinocyte inducing effect of the 4mer and the L3mer is produced via TLR4.

## Claims

1. A sirtuin inducer, **characterized by** containing as an active ingredient a hyaluronic acid fragment selected from sizes of 2 to 20 sugar units, a pharmaceutically acceptable salt thereof, or a pharmaceutically acceptable solvate thereof.

2. The sirtuin inducer according to Claim 1, wherein the hyaluronic acid fragment is selected from sizes of 3 to 10 sugar units.

3. The sirtuin inducer according to Claim 1, wherein the hyaluronic acid fragment is selected from a size of 3 or 4 sugar units.

4. The sirtuin inducer according to any of Claims 1 to 3, for use in treatment and/or prevention of heart disease, arterial sclerosis, osteoporosis, inflammatory bowel disease, dementia, apoplexy, metabolic syndrome, cancer, pulmonary disease, renal disease, diabetes, osteoarthritis, rheumatism, progeria, radiation injury, muscle disorder, brain development disorder, neurological illness, hypertension, or obesity, or to inhibit aging.

5. The sirtuin inducer according to any of Claims 1 to 3, employed in the form of a pharmaceutical, functional food product, or cosmetic.

6. A tissue repairing agent, **characterized by** containing as an active ingredient a hyaluronic acid fragment selected from sizes of 2 to 20 sugar units, a pharmaceutically acceptable salt thereof, or a pharmaceutically acceptable solvate thereof.

7. The tissue repairing agent according to Claim 6,
wherein the hyaluronic acid fragment is selected from sizes of 3 to 10 sugar units.

8. The tissue repairing agent according to Claim 6,
wherein the hyaluronic acid fragment is selected from a size of 3 or 4 sugar units.

9. The tissue repairing agent according to any of Claims 6 to 8, for use in repair of the cornea, dermis, epithelial tissue, neurological tissue, or cardiac muscle tissue.

10. The tissue repairing agent according to any of Claims 6 to 8, employed in the form of a pharmaceutical, functional food product, or cosmetic.

11. A hepatocyte growth factor inducer **characterized by** containing as an active ingredient a hyaluronic acid fragment selected from sizes of 2 to 20 sugar units, a pharmaceutically acceptable salt thereof, or a pharmaceutically acceptable solvate thereof.

12. The hepatocyte growth factor inducer according to Claim 11, wherein the hyaluronic acid fragment is selected from sizes of 3 to 10 sugar units.

13. The hepatocyte growth factor inducer according to Claim 11, wherein the hyaluronic acid fragment is selected from a size of 3 or 4 sugar units.

14. The hepatocyte growth factor inducer according to any of Claims 11 to 13, for use in treatment and/or prevention of liver damage, or in organ preservation.

15. The hepatocyte growth factor inducer according to any of Claims 11 to 13, employed in the form of a pharmaceutical, functional food product, or cosmetic.

16. A tissue homeostasis maintenance agent, **characterized by** containing as an active ingredient a hyaluronic acid fragment selected from sizes of 2 to 20 sugar units, a pharmaceutically acceptable salt thereof, or a pharmaceutically acceptable solvate thereof.

17. The tissue homeostasis maintenance agent according to Claim 16, wherein the hyaluronic acid fragment is selected from sizes of 3 to 10 sugar units.

18. The tissue homeostasis maintenance agent according to Claim 16, wherein the hyaluronic acid fragment is selected from a size of 3 or 4 sugar units.

19. The tissue homeostasis maintenance agent according to any of Claims 16 to 18, for use in maintaining stemness and inducing differentiation of cells in tissue.

20. The tissue homeostasis maintenance agent according to any of Claims 16 to 18, employed in the form of a pharmaceutical, functional food product, or cosmetic.

21. A TLR4 agonist, **characterized by** containing as an active ingredient a hyaluronic acid fragment selected from sizes of 2 to 20 sugar units, a pharmaceutically acceptable salt thereof, or a pharmaceutically acceptable solvate thereof.

22. The TLR4 agonist according to Claim 21, wherein the hyaluronic acid fragment is selected from sizes of 3 to 10 sugar units.

23. The TLR4 agonist according to Claim 21, wherein the hyaluronic acid fragment is selected from a size of 3 or 4 sugar units.

24. The TLR4 agonist according to any of Claims 21 to 23, for use in treatment and/or prevention of disease in the cornea, dermis, liver, neurological tissue, kidney, lung, heart, skeletal muscles, pancreas, or blood vessels.

25. The TLR4 agonist according to any of Claims 21 to 23, employed in the form of a pharmaceutical, functional food product, or cosmetic.
